# EUROPEAN PATENT APPLICATION

(11) **EP 4 645 265 A1**
(43) Date of publication of application: **05.11.2025**
(21) Application number: 23919553.0
(22) Date of filing: 28.12.2023
(51) Int. Cl.: G06V 20/69

(54) **BASE CALLING METHOD AND APPARATUS, ELECTRONIC DEVICE, AND STORAGE MEDIUM**

(30) Priority: 31.01.2023 CN 202310090985
(71) Applicant: GeneMind Biosciences Company Limited, Shenzhen, Guangdong 518000 (CN)
(72) Inventor: CHEN, Weiyue, Shenzhen, Guangdong 518000 (CN); LIU, Yurun, Shenzhen, Guangdong 518000 (CN)
(74) Representative: Michalski Hüttermann & Partner Patentanwälte mbB
(86) International application number: PCT/CN2023/142603
(87) International publication number: WO 2024/159993

(57) **Abstract**

The present application discloses a base calling method and apparatus, an electronic device, and a storage medium. The method for base calling includes: determining a first sequencing information based on intensity features of a first spot in images corresponding to a consecutive cycles of base extension reactions including a designated cycle of base extension reaction, where a is a natural number greater than or equal to 1; and determining a base type of the designated cycle of base extension reaction based on the first sequencing information and a basecall model, where the basecall model is determined based on a second sequencing information corresponding to the a consecutive cycles of base extension reactions in a training sample and base type of at least one cycle of base extension reaction in the a consecutive cycles of base extension reactions, and the second sequencing information includes the first sequencing information. The technical schemes of the examples of the present application improve the accuracy and efficiency of base calling.

## Description

The present application claims priority to the Chinese Patent Application No. 202310090985.0 entitled "METHOD AND APPARATUS FOR BASE CALLING, ELECTRONIC DEVICE, AND STORAGE MEDIUM" filed with the Chinese Patent Office on January 31, 2023, which is incorporated herein by reference in its entirety.

### TECHNICAL FIELD

The present application relates to the field of bioinformatics technology, and in particular, to a base calling method and apparatus, an electronic device, and a storage medium.

### BACKGROUND

To overcome the disadvantages of low cost-efficiency and throughput of the first-generation sequencing technology, the next-generation sequencing (NGS) technology, which is capable of performing parallel sequencing on a large quantity of nucleic acid molecules at a time, has been developed. Existing next-generation sequencing techniques, as represented by the bridge amplification technique of Illumina and the DNBSEQ technique of MGI, achieve the identification and distinction of 4 bases (A, C, G, and T/U) in a nucleic acid molecule sequence by detecting a fluorescence signal. Taking the widely used sequencing-by-synthesis (SBS) technique of ILLUMINA as an example, the method uses dNTPs (dATP, dCTP, dGTP, and dTTP/dUTP) with a fluorescence label and a blocking group, and dATP, dCTP, dGTP, and dTTP/dUTP each carry a different fluorescent label group. Due to the presence of the blocking group, only one dNTP complementary to the nucleic acid molecule template is added to each nucleic acid molecule template in each cycle of polymerization reaction, and the type of the dNTP added to each nucleic acid molecule template in the cycle can be detected after the excitation with an excitation light with a corresponding wave band. The blocking group and the fluorescent label group are then cleaved with appropriate chemical agents to allow the sequencing reaction to proceed normally to the next cycle.

With the wide application of sequencing techniques, the next-generation sequencing is challenged by low base calling accuracy in some sequencing application fields, leaving unmet demands. Therefore, there's a need for a method for base calling to solve the above technical problems.

### SUMMARY

In order to solve the above technical problems, the examples of the present application provide a base calling method and apparatus, an electronic device, and a storage medium.

In a first aspect, the examples of the present application provide a method for base calling, including:
determining a first sequencing information based on intensity features of a first spot in images corresponding to a consecutive cycles of base extension reactions including a designated cycle of base extension reaction, where a is a natural number greater than or equal to 1; and
determining a base type of the designated cycle of base extension reaction based on the first sequencing information and a basecall model, where the basecall model is determined based on a second sequencing information corresponding to the a consecutive cycles of base extension reactions in a training sample and base type of at least one cycle of base extension reaction in the a consecutive cycles of base extension reactions, and the second sequencing information includes the first sequencing information.

In a second aspect, the examples of the present application provide an apparatus for base calling, including:
a first sequencing information confirmation module, configured for determining a first sequencing information based on intensity features of a first spot in images corresponding to a consecutive cycles of base extension reactions including a designated cycle of base extension reaction, where a is a natural number greater than or equal to 1; and
a designated-cycle base type confirmation module, configured for determining a base type of the designated cycle of base extension reaction based on the first sequencing information and a basecall model, where the basecall model is determined based on a second sequencing information corresponding to the a consecutive cycles of base extension reactions in a training sample and base type of at least one cycle of base extension reaction in the a consecutive cycles of base extension reactions, and the second sequencing information includes the first sequencing information.

In a third aspect, the examples of the present application provide an electronic device, including:
one or more processors; and
a storage apparatus, configured for storing one or more programs, where
when the one or more programs are executed by the one or more processors, the one or more processors implement the method for base calling according to any one of the examples of the present application.

In a fourth aspect, the examples of the present application provide a storage medium including a computer-executable instruction, where the computer-executable instruction is configured for executing the method for base calling according to any one of the examples of the present application when executed by a computer processor.

According to the technical schemes of the examples of the present application, the first sequencing information is determined through the intensity features of the first spot in the images corresponding to the a consecutive cycles of base extension reactions including the designated cycle of base extension reaction, and the base type of the designated cycle is obtained according to the first sequencing information and the basecall model. The first sequencing information is determined according to the intensity features of the combined bases, such that the information of the base in the designated cycle in the first sequencing information is more comprehensive, and the base type of the designated cycle can be determined according to the basecall model and the first sequencing information, thus helping more accurate identification of the types and the sequence of the bases binding to the template nucleic acid in the base extension reaction and improving the sequencing accuracy.

### BRIEF DESCRIPTION OF THE DRAWINGS

In order to more clearly illustrate the technical schemes in the examples of the present application or the prior art, the drawings required for use in the description of the examples or the prior art will be briefly described below. It is obvious that the drawings in the description below are only some examples of the present application, and other drawings can be derived from these drawings by those of ordinary skill in the art without creative efforts.

Among the drawings:
FIG. 1 is a schematic flowchart illustrating a method for base calling according to one example;
FIG. 2 is a schematic flowchart illustrating a method for base calling according to one example;
FIG. 3 is a schematic flowchart illustrating a method for base calling according to one example;
FIG. 4 is a diagram illustrating the comparison of error counts by error types for sequencing a human genome sample using a two-base machine learning model and an original base calling algorithm according to one example;
FIG. 5 is a diagram illustrating the comparison of error counts by error types for sequencing an *Escherichia coli* sample using a two-base machine learning model and an original base calling algorithm according to one example;
FIG. 6 is a schematic of an apparatus for base calling according to one example; and
FIG. 7 is a schematic of an electronic device according to one example.

### DETAILED DESCRIPTION

The technical schemes in the examples of the present application will be described below clearly and comprehensively in conjunction with the drawings in the examples of the present application. It is obvious that the described examples are part of the examples of the present application, but not all of them. On the basis of the examples of the present application, all other examples obtained by those of ordinary skill in the art without creative efforts shall fall within the claimed scope of the present application.

In the description herein, the terms "first", "second", and the like are used for illustrative purposes only, and should not be construed as indicating or implying relative importance or implicitly indicating the number or sequence of indicated technical features. In the description of the present application, unless otherwise specifically defined, "a plurality of" means two or more than two.

In the description herein, the term sequencing, also known as sequence determination or gene sequencing, refers to the determination of a nucleotide sequence in a nucleic acid molecule, including DNA sequencing and/or RNA sequencing. The sequencing of the present application does not define the length of the nucleic acid sample of interest, i.e., the nucleic acid molecule template, and includes long fragment sequencing and/or short fragment sequencing. The sequencing may be the identification of the types and the determination of the sequence of bases in multiple continuous or non-continuous specific positions of a nucleic acid sequence. Sequencing methods include sequencing-by-synthesis (SBS) or sequencing-by-ligation (SBL), including a process in which a nucleotide or nucleotide analogue binds to a template, i.e., base extension reaction.

The sequencing generally involves multiple cycles of process to achieve the determination of the types and sequence of multiple bases or nucleotides on the nucleic acid template. The examples of the present application refer to each cycle of the "process to achieve the determination of the types and sequence of multiple bases or nucleotides on the nucleic acid template" as one "cycle of sequencing". The "cycle of sequencing", also known as "sequencing cycle", may be defined as the completion of one base extension of the four types of nucleotides/bases; in other words, one "cycle of sequencing" may be defined as the determination of the base or nucleotide type at any given position on the template. For sequencing platforms that achieve sequencing on the basis of polymerization or ligation reactions, one cycle of sequencing includes the process of binding four types of nucleotides (including nucleotide analogs) to the nucleic acid template at a time according to the base complementary rule and acquiring the corresponding signals emitted. For platforms that achieve sequencing on the basis of the polymerization reaction, a reaction system includes reaction substrate nucleotides, polymerase, and a nucleic acid template. A sequence fragment (a sequencing primer) binds to the nucleic acid template, and on the basis of the base pairing rules and the principle of polymerization reaction, the added reaction substrate nucleotides are linked to the sequencing primer under the catalysis of the polymerase to achieve the binding of the nucleotide to a specific position on the nucleic acid template. Generally, one cycle of sequencing may include one or more base extensions (repeats). For example, four types of nucleotides are sequentially added to the reaction system to each perform base extensions and corresponding acquisition of reaction signals, and one cycle of sequencing includes four base extensions; for another example, four types of nucleotides are added into the reaction system in any combinations (such as in pairs or in one-three combinations), the two combinations each perform base extensions and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension.

In the description herein, images acquired from sequencing reaction/base extension reaction or images obtained by conversion or construction based on these images may be grayscale images or colored images. For a grayscale image, the pixel value refers to the grayscale value; for a 16-bit grayscale image such as tiff grayscale image, the pixel value ranges from 0 to 65535; for an 8-bit grayscale image, the pixel value ranges from 0 to 255. For a colored image, each pixel has three pixel values. An image detection/target information identification may be performed directly based on an array of the pixel values using a provided method and/or system; alternatively, the colored image can be converted into a grayscale image first, which is then processed and subjected to information identification, thereby reducing the calculation and complexity in the image detection and signal identification processes. A non-grayscale image may be converted into a grayscale image with methods including but not limited to floating point algorithm, integer method, shift method, mean value method, etc.

In the description herein, unless otherwise specified, based on the image information, the terms "intensity" and pixel (pixel value) are used interchangeably. The intensity or pixel value may be a real or objective absolute value, or may be a relative value including various variants based on the real pixel value, such as an increased pixel value, a decreased pixel value, a proportion or relationship based on the pixel value. Generally, when the comparison between a plurality of images or spots or positions in intensity/pixel size is involved, the intensity/pixel value of the images or spots or positions is the intensity/pixel size after the same processing, such as objective pixel values or pixel values after the same transformation; when the comparison and analysis based on the information of particular positions in one or more images are involved and the particular positions are determined, the images are preferably aligned and kept in the same coordinate system. In one embodiment, the "intensity" noted in the examples of the present application may be "fluorescence intensity".

In the description herein, the term "spot" or "peak" refers to luminous spots or points in an image, and one luminous spot occupies at least one pixel. The determination of the "spot" is the determination of an optical signal from an extended base or base cluster.

In the description herein, A represents adenine and may also represent adenine nucleotide or an analog thereof; C represents cytosine and may also represent cytosine nucleotide or an analog thereof; G represents guanine and may also represent guanine nucleotide or an analog thereof; T represents thymine and may also represent thymine nucleotide or an analog thereof; U represents uracil and may also represent uracil nucleotide or an analog thereof. It will be appreciated that the representations of A, C, G, and T/U are consistent in the examples of the present application. When one of them represents a base, the other three also represent bases. For example, when A represents adenine, correspondingly, C represents cytosine, G represents guanine, T represents thymine/U represents uracil. When one of them represents a nucleotide or an analog thereof, the other three also represent nucleotides or analogs thereof. For example, when A can represent adenine nucleotide or an analog thereof, correspondingly, C represents cytosine nucleotide or an analog thereof, G represents guanine nucleotide or an analog thereof, T represents thymine nucleotide or an analog thereof/U represents uracil or an analog thereof. The "/" in T/U means "or". That is, "T/U" means "T or U".

In a platform for determining a nucleic acid sequence based on optical imaging, after an image is acquired, for base calling, spots in the acquired image will be first detected and identified; that is, real signals from extended bases or base clusters will be detected and identified. Then each of the spots will be aligned with spots on a sequencing template. For example, the spots in the image of interest are traversed, and if a certain spot in the image of interest and a spot on the sequencing template are close enough (which is related to the resolution and the like), the two spots are considered to overlap, a nucleic acid molecule of interest is considered present in the position corresponding to the spot in the image of interest (the spot is considered as a valid spot), and the nucleic acid molecule of interest is involved in nucleotide binding reaction (base extension reaction), thereby identifying the type of the nucleotide/base binding to the nucleic acid molecule of interest. In this method for base calling, whether the nucleic acid molecule represented on the sequencing template undergoes a base extension reaction in the current cycle is determined by comparing the distance between the coordinates of the spot in the image of interest and the coordinates of the spot on the sequencing template; if it undergoes a reaction, the type of the base added is read or the type of the base corresponding to the nucleic acid molecule is read. It is found that this method is readily influenced by image quality, spot positioning algorithm, spot density distribution and the like, and is prone to base calling errors.

In the base calling method, the intensity feature determines the base type identified in one cycle of base extension reaction. Therefore, the brightness and count of spots of each cycle are obtained by a base calling software from the images, and various derivative influence factors such as the ratio of the greatest brightness to the second greatest brightness are established on the basis of the brightness and count of the spots. Then a machine learning model is established on this basis to correct various errors, so as to reduce the mode errors and the probability of false positives and improve the accuracy of the instrument and the speed of bioinformatic analysis.

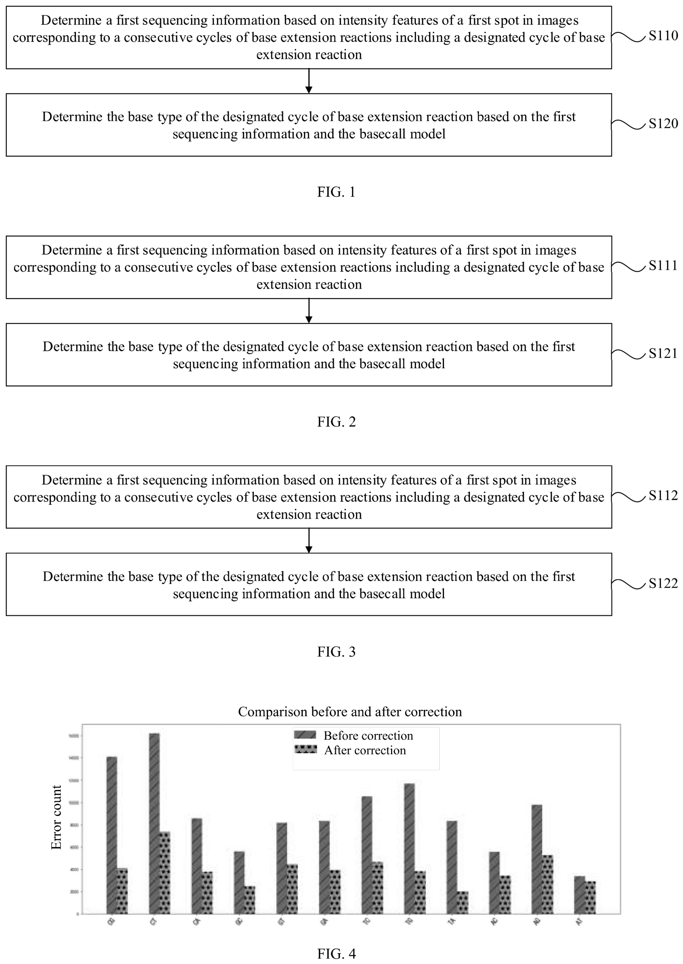

As such, the examples of the present application provide a method for base calling. The method for base calling according to the examples of the present application is applicable to the instance of identifying the type of the base introduced in a base extension reaction in a sequencing process. The method may be executed by an apparatus for base calling. The apparatus may be achieved in the form of software and/or hardware. FIG. 2 illustrates a schematic flowchart of the method for base calling provided in the examples of the present application.

As shown in FIG. 1, the method for base calling according to the examples of the present application includes: S110, determining a first sequencing information based on intensity features of a first spot in images corresponding to a consecutive cycles of base extension reactions including a designated cycle of base extension reaction.

In the examples of the present application, the base of the designated cycle refers to the base binding to the nucleic acid molecule of interest during the cycle of base extension reaction requiring base calling or base correction during multiple cycles of base extension reactions of the nucleic acid molecule of interest. Correspondingly, the base of the designated cycle of extension reaction refers to the base binding to the nucleic acid molecule of interest during the designated cycle of base extension reaction. Hereinafter, the "n preceding cycles of base extension reactions" refers to the n cycles of base extension reactions occurring before the designated cycle of base extension reaction, and correspondingly, the "bases of the n preceding cycles" refers to the bases binding to the nucleic acid molecule of interest in the n preceding cycles of base extension reactions. Similarly, the "m succeeding cycles of base extension reactions" refers to the m cycles of base extension reactions occurring before the designated cycle of base extension reaction, and correspondingly, the "bases of the m succeeding cycles" refers to the bases binding to the nucleic acid molecule of interest in the m succeeding cycles of base extension reactions.

In one embodiment, the first sequencing information for base calling in the designated cycle of base extension reaction includes the intensity feature of the first spot in the image corresponding to the designated cycle of base extension reaction. In this case, the base type of the designated cycle of base extension reaction is determined by a preset basecall model and the first sequencing information determined by the intensity feature of the first spot in the image corresponding to the designated cycle of base extension reaction. The image may be a fluorescent image.

In another embodiment, the first sequencing information for base calling in the designated cycle of base extension reaction is derived not only from the intensity feature of the first spot in the image corresponding to the designated cycle of base extension reaction, but also from the intensity features of the first spot in the image corresponding to one or more cycles of base extension reactions before and after the designated cycle of base extension reaction, i.e., the intensity features of the first spot in the images corresponding to the a consecutive cycles of base extension reactions including the designated cycle of base extension reaction. In some examples, the a consecutive cycles of base extension reactions include the designated cycle of base extension reaction requiring base calling, and at least one of the n preceding cycles of base extension reactions and the m succeeding cycles of base extension reactions. The n preceding cycles of base extension reactions are the n cycles of base extension reaction occurring before the designated cycle of base extension reaction; the m succeeding cycles of base extension reactions are the m cycles of base extension reactions occurring after the designated cycle of base extension reaction.

The values of m and n satisfy the following conditions: n is an integer greater than or equal to 0, and m is an integer greater than or equal to 0. When both m and n are 0, the first implementation described above prevails. When at least one of m and n is not 0, the second implementation described above prevails. Illustratively, at least one of m and n being not 0 includes the following cases: m is 0 while n is not 0; m is not 0 while n is 0; m is not 0 and n is not 0. It will be appreciated that the values of m and n may also satisfy: m + n + 1 = a, a being a natural number greater than or equal to 1.

In some examples, m and n are each selected from a natural number from 1 to 5. In such a case, the intensity features of the images corresponding to the base extension reactions can be increased by increasing the number of cycles of base extension reactions, so as to increase the number of data sources of the first sequencing information, which is beneficial to improving the identification accuracy of the base type of the designated cycle of base extension reaction, and particularly to reducing the base calling error rate associated with the base combination type. Illustratively, m may be selected from 1, 2, 3, 4, 5, etc., and n may be selected from 1, 2, 3, 4, 5, etc. It will be appreciated that the values of m and n are not limited thereto. Theoretically, greater values of m and n may result in more acquired features and more generated data, which may be advantageous for reducing the error rate of base calling, but complicated processing and reduced efficiency for base calling due to the increased data volume.

In one implementation, the a consecutive cycles of base extension reactions include the designated cycle of base extension reaction and the n preceding cycles of base extension reactions. The first sequencing information obtained based on the images corresponding to the a consecutive cycles of base extension reactions includes intensity features of spots in the image obtained from the designated cycle of base extension reaction and intensity features of spots in the images obtained from the n preceding cycles of base extension reactions, and other features associated with intensity features of spots in the images corresponding to the designated cycle of base extension reaction and the n preceding cycles of base extension reactions as a whole.

In another implementation, the a consecutive cycles of base extension reactions include a combination of the designated cycle of base extension reaction and the m succeeding cycles of base extension reactions. The first sequencing information obtained based on the base combination includes the sequencing information of the base of the designated cycle and the m succeeding cycles of base extension reactions. The first sequencing information obtained based on the images corresponding to the a consecutive cycles of base extension reactions includes intensity features of spots in the image obtained from the designated cycle of base extension reaction and intensity features of spots in the images obtained from the m succeeding cycles of base extension reactions, and other features associated with intensity features of spots in the images corresponding to the designated cycle of base extension reaction and the m succeeding cycles of base extension reactions as a whole.

In still another implementation, the a consecutive cycles of base extension reactions include a combination of the designated cycle of base extension reaction, the n preceding cycles of base extension reactions, and the m succeeding cycles of base extension reactions. The first sequencing information obtained based on the images corresponding to the a consecutive cycles of base extension reactions includes the intensity features of spots in the image obtained from the designated cycle of base extension reaction, intensity features of spots in the images obtained from the n preceding cycles of base extension reactions and intensity features of spots in the images obtained from the m succeeding cycles of base extension reactions, other features associated with intensity features of spots in the images corresponding to the designated cycle of base extension reaction and the m succeeding cycles of base extension reactions as a whole, other features associated with intensity features of spots in the images corresponding to the designated cycle of base extension reaction and the n preceding cycles of base extension reactions as a whole, and other features associated with intensity features of spots in the images corresponding to the designated cycle of base extension reaction, the n preceding cycles of base extension reactions and the m succeeding cycles of base extension reactions as a whole.

The first sequencing information is an information associated with the intensity features of the first spot in the images corresponding to the a consecutive cycles of base extension reactions, and is used for identifying the type of the base binding to the nucleic acid molecule of interest in the designated cycle of base extension reaction. It will be appreciated that the base calling described in the examples of the present application is applicable to the whole biochip, and when sequencing is performed on the nucleic acid molecules of interest on the biochip, the base calling is achieved on one or more, even all, of the nucleic acid molecules of interest. Specifically, the first sequencing information is determined based on the intensity features of the first spot in the images corresponding to the a consecutive cycles of base extension reactions including the designated cycle of base extension reaction, such that the intensity features of the spots in the images corresponding to the a consecutive cycles of base extension reactions and the like are included in the first sequencing information, thereby improving the comprehensiveness of the sequencing information. The images corresponding to the a consecutive cycles of base extension reactions include the image corresponding to each cycle of base extension reaction, and each cycle of base extension reaction includes the images acquired from the four channels of an A base channel, a T/U base channel, a C base channel, and a G base channel.

In some embodiments, before the first sequencing information is determined based on the intensity features of the first spot in the images corresponding to the a consecutive cycles of base extension reactions including the designated cycle of base extension reaction, the intensity features of the first spot in the images corresponding to the a consecutive cycles of base extension reactions are determined.

In one example, determining the intensity features of the first spot in the images includes:
(1) mapping the coordinates of second spots in a spot set of the sequencing template to each of the images, so as to determine the first spot in the images.

In this step, the sequencing template is a template constructed based on a biochip under test with the nucleic acid molecules of interest and is used for mapping the positions of the nucleic acid molecules of interest in the biochip in each cycle of base extension reaction. The template includes the spot set consisting of second spots with specific coordinates, and each of the second spots in the spot set corresponds to one nucleic acid molecule of interest in the biochip. In the examples of the present application, the sequencing template may be constructed during the base calling on the biochip, or may be constructed in advance of the base calling. In one example, the spot set corresponding to the sequencing template is constructed in advance based on images and then saved for later use.

In this example, mapping the coordinates of the second spots in the spot set of the sequencing template to each of the images, so as to confirm the first spot in the images, includes: mapping the coordinates of each second spot in the spot set of the sequencing template to the image, determining the position corresponding to the coordinates of the second spot in the image, and determining the spot corresponding to the position as the first spot. Illustratively, this can be achieved by the following means: aligning each second spot in the spot set of the sequencing template to the image with each spot in the image of interest. For example, the spots in the image of interest are traversed, and if a certain spot in the image of interest and the second spot on the sequencing template are close enough (which is related to the resolution and the like), the two spots are considered to overlap, a nucleic acid molecule of interest is considered present in the position corresponding to the spot in the image of interest (the spot is considered as a valid spot), and the nucleic acid molecule of interest is involved in nucleotide binding reaction (base extension reaction). The spot is thus determined as the first spot. That is, the first spots are spots corresponding to the coordinates of the second spots in the spot set of the sequencing template among all spots in the optical image.

(2) determining the intensity features of the first spot in the images corresponding to the a consecutive cycles of base extension reactions based on the first spot in each of the images.

According to the technical schemes of the examples of the present application, the intensity features of the first spot in the images corresponding to the a consecutive cycles of base extension reactions including the designated cycle of base extension reaction can be obtained according to the first spot (i.e., the valid spot) in the image, thus improving the accuracy of the intensity features.

In the examples of the present application, the intensity feature includes any one of an initial intensity and a corrected intensity. The initial intensity refers to the intensity directly determined from the image, i.e., the original intensity obtained according to the first spot or valid spot. The corrected intensity refers to an intensity obtained by correcting the initial intensity with a predetermined correction manner. The predetermined correction manner includes, but is not limited to, background correction, crosstalk correction, and reaction asynchrony correction.

In one example, the intensity feature is an initial intensity feature. Illustratively, the method for confirming the intensity features of the first spot in the images corresponding to the a consecutive cycles of base extension reactions includes: acquiring the images obtained from the a consecutive cycles of base extension reactions, extracting original intensities of the A base channel, the C base channel, the G base channel, and the T/U base channel at the first spot in the images, and confirming that the original intensities of the A base channel, the C base channel, the G base channel, and the T/U base channel are the initial intensities of the A base channel, the C base channel, the G base channel, and the T/U base channel at the first spot, respectively.

In one example, the intensity feature is a corrected intensity feature. After acquiring the images obtained from the a consecutive cycles of base extension reactions, the original intensities of the A base channel, the C base channel, the G base channel, and the T/U base channel at the first spot in the images are extracted, and the original intensities of the A base channel, the C base channel, the G base channel, and the T/U base channel are determined as the initial intensities of the A base channel, the C base channel, the G base channel, and the T/U base channel at the first spot, respectively; at least one of the background correction, crosstalk correction, and reaction asynchrony correction is performed on the initial intensities to determine the corrected intensity. Illustratively, the method for confirming the intensity features of the first spot in the images corresponding to the a consecutive cycles of base extension reactions includes: acquiring the images obtained from the a consecutive cycles of base extension reactions, extracting original intensities of the A base channel, the C base channel, the G base channel, and the T/U base channel at the first spot in the images, and confirming that the original intensities of the A base channel, the C base channel, the G base channel, and the T/U base channel are the initial intensities of the A base channel, the C base channel, the G base channel, and the T/U base channel at the first spot, respectively; at least one of the background correction, crosstalk correction, and reaction asynchrony correction is performed based on the initial intensities to confirm the corrected intensity. In this example, the corrected intensity feature is used as the source of the first sequencing information, which eliminates interferences caused by factors such as background, crosstalk, and reaction asynchrony, thereby enhancing the purity of the sequencing signal. When the first sequencing information is used as a feature value and inputted into the corresponding basecall model, it is more conducive to improving the accuracy of base calling.

It will be appreciated that the intensity feature used as the source of the first sequencing information is consistent with the intensity feature used as the source of the second sequencing information when the basecall model is established. That is, when the intensity feature used as the source of the first sequencing information is an initial intensity feature, the intensity feature used as the source of the second sequencing information is also an initial intensity feature; when the intensity feature used as the source of the first sequencing information is a corrected intensity feature, the intensity feature used as the source of the second sequencing information is also a corrected intensity feature, and the correction manners selected for correcting intensity features are consistent.

In the examples of the present application, the first sequencing information is determined based on the intensity features of the first spot in the images corresponding to the a consecutive cycles of base extension reactions including the designated cycle of base extension reaction. The first sequencing information is the sequencing information corresponding to the a consecutive cycles of base extension reactions when sequencing the nucleic acid molecule of interest, and the sequencing information is an sequencing information obtained based on the intensity feature of the first spot in the acquired image.

In some examples, the first sequencing information includes at least one of the following features or a combination thereof, or a feature formed by dimensionality reduction for a combination of more than one of the following features: the ratio of an intensity corresponding to a base channel with the maximum brightness to the total intensity in the four base channels at the coordinate position corresponding to the first spot in the image acquired in the designated cycle of base extension reaction; the total number of the first spots in the image acquired in the designated cycle of base extension reaction; the intensity feature of the A base channel at the coordinate position corresponding to the first spot in the image acquired in the designated cycle of base extension reaction; the intensity feature of the A base channel at the coordinate position corresponding to the first spot in the image acquired in each cycle of base extension reaction from the 1^{st} preceding cycle to the n^{th} preceding cycle of base extension reactions; the intensity feature of the A base channel at the coordinate position corresponding to the first spot in the image acquired in each cycle of base extension reaction from the 1^{st} succeeding cycle to the m^{th} succeeding cycle of base extension reactions; the intensity feature of the C base channel at the coordinate position corresponding to the first spot in the image acquired in the designated cycle of base extension reaction; the intensity feature of the C base channel at the coordinate position corresponding to the first spot in the image acquired in each cycle of base extension reaction from the 1^{st} preceding cycle to the n^{th} preceding cycle of base extension reactions; the intensity feature of the C base channel at the coordinate position corresponding to the first spot in the image acquired in each cycle of base extension reaction from the 1^{st} succeeding cycle to the m^{th} succeeding cycle of base extension reactions; the intensity feature of the G base channel at the coordinate position corresponding to the first spot in the image acquired in the designated cycle of base extension reaction; the intensity feature of the G base channel at the coordinate position corresponding to the first spot in the image acquired in each cycle of base extension reaction from the 1^{st} preceding cycle to the n^{th} preceding cycle of base extension reactions; the intensity feature of the G base channel at the coordinate position corresponding to the first spot in the image acquired in each cycle of base extension reaction from the 1^{st} succeeding cycle to the m^{th} succeeding cycle of base extension reactions; the intensity feature of the T/U base channel at the coordinate position corresponding to the first spot in the image acquired in the designated cycle of base extension reaction; the intensity feature of the T/U base channel at the coordinate position corresponding to the first spot in the image acquired in each cycle of base extension reaction from the 1^{st} preceding cycle to the n^{th} preceding cycle of base extension reactions; the intensity feature of the T/U base channel at the coordinate position corresponding to the first spot in the image acquired in each cycle of base extension reaction from the 1^{st} succeeding cycle to the m^{th} succeeding cycle of base extension reactions; the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and the standard deviation of the intensities corresponding to the four base channels at the coordinate position corresponding to the first spot in the image acquired in the designated cycle of base extension reaction.

S120, determining the base type of the designated cycle of base extension reaction based on the first sequencing information and the basecall model.

In this step, the basecall model is determined based on a second sequencing information corresponding to the a consecutive cycles of base extension reactions in a training sample and base type of at least one cycle of base extension reaction in the a consecutive cycles of base extension reactions, and the second sequencing information includes the first sequencing information.

Specifically, the first sequencing information is processed via the basecall model to give the base type of the designated cycle.

According to the technical schemes of the examples of the present application, the first sequencing information is determined through the intensity features of the first spot in the images corresponding to the a consecutive cycles of base extension reactions, and the base type of the designated cycle is obtained according to the first sequencing information and the basecall model. Since the first sequencing information is determined according to the intensity features corresponding to the a consecutive cycles of base extension reactions, the sequencing information corresponding to the designated cycle of base extension reaction in the first sequencing information is more comprehensive, and the base type of the designated cycle can be obtained according to the basecall model and the first sequencing information, thus making the base type of the designated cycle more accurate. Therefore, the identified base type of the base extension reaction can be corrected, and the base type of the designated cycle that is undergoing the base extension reaction can be directly subjected to base calling, thereby reducing the error rate of base calling.

In some examples, determining the base type of the designated cycle of base extension reaction based on the first sequencing information and the basecall model, includes:
(1) determining a base combination type corresponding to the a consecutive cycles of base extension reactions. In this step, the base combination type corresponding to the a consecutive cycles of base extension reactions refers to the form of the consecutive base combination corresponding to the a consecutive cycles of base extension reactions. In the examples of the present application, the base combination type includes at least the base corresponding to the base extension reaction requiring base calling (hereinafter referred to as the "base of the designated cycle"), and may further include at least one of the bases corresponding to the n preceding cycles of base extension reactions (hereinafter referred to as the "bases of the n preceding cycles") and the bases corresponding to the m succeeding cycles of base extension reactions (hereinafter referred to as the "bases of the m succeeding cycles"), where the bases corresponding to multiple consecutive base extension reactions form the base combination.

In some examples, the a consecutive cycles of base extension reactions include the designated cycle of base extension reaction requiring base calling and the n preceding cycles of base extension reactions. Correspondingly, the combined bases formed by the a consecutive cycles of base extension reactions include the base of the designated cycle and the bases of the n preceding cycles, and the combination type refers to the combination form of the base of the designated cycle and the bases of the n preceding cycles, e.g., the combination form of the base of the designated cycle and the base of the preceding cycle, the combination form of the base of the designated cycle and the bases of the two preceding cycles, and the like. n is a natural number greater than or equal to 1. Optionally, in the examples of the present application, n is less than or equal to 5. Optionally, n is 1, 2, or 3.

In some examples, the a consecutive cycles of base extension reactions include the designated cycle of base extension reaction requiring base calling and the m succeeding cycles of base extension reactions. Correspondingly, the combined bases formed by the a consecutive cycles of base extension reactions include the base of the designated cycle and the bases of the m succeeding cycles, and the combination type refers to the combination form of the base of the designated cycle and the bases of the m succeeding cycles, e.g., the combination form of the base of the designated cycle and the base of the succeeding cycle, the combination form of the base of the designated cycle and the bases of the two succeeding cycles, and the like. m is a natural number greater than or equal to 1. Optionally, in the examples of the present application, m is less than or equal to 5. Optionally, m is 1, 2, or 3.

In some examples, the a consecutive cycles of base extension reactions include the designated cycle of base extension reaction requiring base calling, the n preceding cycles of base extension reactions, and the m succeeding cycles of base extension reactions. Correspondingly, the combined bases formed by the a consecutive cycles of base extension reactions include the base of the designated cycle, the bases of the n preceding cycles, and the bases of the m succeeding cycles, and the combination type refers to the combination form of the base of the designated cycle, the bases of the n preceding cycles, and the bases of the m succeeding cycles, e.g., the combination form of the base of the designated cycle, the base of the preceding cycle and the base of the succeeding cycle, the combination form of the base of the designated cycle, the bases of the two preceding cycles and the bases of the two succeeding cycles, and the like. m is a natural number greater than or equal to 1. Optionally, in the examples of the present application, m is less than or equal to 5, and n is less than or equal to 5. Optionally, m is 1, 2, or 3, and n is 1, 2, or 3.

Illustratively, when the a consecutive cycles of base extension reactions include the designated cycle of base extension reaction, the preceding cycle of base extension reaction, and the succeeding cycle of base extension reaction, the corresponding base combination type is a combination form of the base of the designated cycle, the base corresponding to the preceding cycle of base extension reaction (hereinafter referred to as the "base of the preceding cycle"), and the base corresponding to the succeeding cycle of base extension reaction (hereinafter referred to as the "base of the succeeding cycle"), i.e., the base combination of base of the preceding cycle-base of the designated cycle-base of the succeeding cycle.

It will be appreciated that the base combination type further includes the instance where only the base corresponding to the designated cycle of base extension reaction is included.

(2) inputting the first sequencing information into the basecall model matching the base combination type, outputting a base combination corresponding to the a consecutive cycles of base extension reactions, and determining the base type of the designated cycle of base extension reaction based on the base combination.

In the step, after the base combination type corresponding to the a consecutive cycles of base extension reactions, a basecall model matching the base combination type is selected. The basecall model matching the base combination type is a basecall model trained based on the features corresponding to a base combination type identical to the base combination type as a sample. Illustratively, when the base combination type is a base combination pattern of base of the preceding cycle-base of the designated cycle-base of the succeeding cycle, the selected basecall model is: a model obtained by training a plurality of training samples based on the features obtained from a three-base combination obtained by three consecutive base extension reactions with the base type of the base in the middle among three bases as the target.

The first sequencing information is input into the basecall model for processing, and the base combination corresponding to the a consecutive cycles of base extension reactions can be output. In this case, the base type of the designated cycle of base extension reaction can be determined based on the base combination and the a consecutive cycles of base extension reactions.

In some examples, the method for establishing the basecall model includes: acquiring a training sample set, where each sample in the training sample set is labeled with a feature value and a target value, the feature value is a second sequencing information determined based on the base combination type, and the target value is the base type corresponding to the designated cycle of base extension reaction in the base combination type; and performing machine learning modeling on each sample in the training sample set based on a specific model structure to give the basecall model.

In the examples of the present application, a training sample set is acquired, that is, a sample labeled with the second sequencing information and the target value is acquired. In this example, the target value is the base type corresponding to the designated cycle of base extension reaction in the examples of the present application in the base combination type. Illustratively, when the a consecutive cycles of base extension reactions include the designated cycle of base extension reaction, the preceding cycle of base extension reaction, and the succeeding cycle of base extension reaction, the corresponding base combination type is the base combination of base of the preceding cycle-base of the designated cycle-base of the succeeding cycle. In this case, the base combination type in the basecall model is a consecutive three-base combination. The feature value is the second sequencing information determined based on the consecutive three-base combination, and the target value is the type of the base in the middle (i.e., the second base) in the consecutive three-base combination.

In the examples of the present application, the second sequencing information includes the first sequencing information. In some examples, the first sequencing information includes at least one of the following features or a combination thereof, or a feature formed by dimensionality reduction for a combination of more than one of the following features: the ratio of an intensity corresponding to a base channel with the maximum brightness to the total intensity in the four base channels at the coordinate position corresponding to the first spot in the image acquired in the base extension reaction corresponding to the target value; the total number of the first spots in the image acquired in the base extension reaction corresponding to the target value; the intensity feature of the A base channel at the coordinate position corresponding to the first spot in the image acquired in the base extension reaction corresponding to the target value; the intensity feature of the A base channel at the coordinate position corresponding to the first spot in the image acquired in each cycle of base extension reaction from the 1^{st} preceding cycle to the n^{th} preceding cycle of base extension reactions; the intensity feature of the A base channel at the coordinate position corresponding to the first spot in the image acquired in each cycle of base extension reaction from the 1^{st} succeeding cycle to the m^{th} succeeding cycle of base extension reactions; the intensity feature of the C base channel at the coordinate position corresponding to the first spot in the image acquired in the base extension reaction corresponding to the target value; the intensity feature of the C base channel at the coordinate position corresponding to the first spot in the image acquired in each cycle of base extension reaction from the 1^{st} preceding cycle to the n^{th} preceding cycle of base extension reactions; the intensity feature of the C base channel at the coordinate position corresponding to the first spot in the image acquired in each cycle of base extension reaction from the 1^{st} succeeding cycle to the m^{th} succeeding cycle of base extension reactions; the intensity feature of the G base channel at the coordinate position corresponding to the first spot in the image acquired in the base extension reaction corresponding to the target value; the intensity feature of the G base channel at the coordinate position corresponding to the first spot in the image acquired in each cycle of base extension reaction from the 1^{st} preceding cycle to the n^{th} preceding cycle of base extension reactions; the intensity feature of the G base channel at the coordinate position corresponding to the first spot in the image acquired in each cycle of base extension reaction from the 1^{st} succeeding cycle to the m^{th} succeeding cycle of base extension reactions; the intensity feature of the T/U base channel at the coordinate position corresponding to the first spot in the image acquired in the base extension reaction corresponding to the target value; the intensity feature of the T/U base channel at the coordinate position corresponding to the first spot in the image acquired in each cycle of base extension reaction from the 1^{st} preceding cycle to the n^{th} preceding cycle of base extension reactions; the intensity feature of the T/U base channel at the coordinate position corresponding to the first spot in the image acquired in each cycle of base extension reaction from the 1^{st} succeeding cycle to the m^{th} succeeding cycle of base extension reactions; the ratio of an intensity corresponding to a base channel with the maximum intensity to an intensity corresponding to a base channel with the second maximum intensity in the four base channels at the coordinate position corresponding to the first spot in the image acquired in the base extension reaction corresponding to the target value; and the standard deviation of the intensities corresponding to the four base channels at the coordinate position corresponding to the first spot in the image acquired in the base extension reaction corresponding to the target value.

Each of the base extension reactions from the 1^{st} preceding cycle to the n^{th} preceding cycle of base extension reactions refers to the 1^{st} cycle to the n^{th} cycle of base extension reactions occurring before the base extension reaction corresponding to the target value, and the 1^{st} succeeding cycle to the m^{th} succeeding cycle of base extension reactions refer to the 1^{st} cycle to the m^{th} cycle of base extension reactions occurring after the base extension reaction corresponding to the target value.

Machine learning modeling is performed on each sample in the training sample set based on a specific model structure to give the basecall model. The schemes of the examples of the present application achieve the establishment of the basecall model, and since the feature value in the training sample is the second sequencing information determined based on the combination type, the second sequencing information is different in the case of a different combination type, such that when the machine learning modeling is performed on samples in the training sample set based on a specific model structure, the obtained basecall models are also different. The basecall model corresponding to each combination type can be obtained by the method according to the examples of the present application.

Optionally, the specific model structure is the lightGMB model. Since the large data volume during the base calling imposes high requirements on the hardware, the cost for base calling is increased. The lightGMB model well processes the classification problems in the case of limited operation RAM of the hardware, and supports the input of classified data, GPU acceleration, and distributed training, thus allowing accelerated processing with limited RAM and providing results with high accuracy. Therefore, when the specific model structure is the lightGMB model, the examples of the present application keep a good balance between the cost-efficiency of the hardware and the accuracy of the output result of the basecall model.

Optionally, the established basecall model may be verified with a test sample set to determine the accuracy and sensitivity of the established basecall model. Optionally, the same data set may be divided into two parts, with one part of the data set used as the training sample set and the other part of the data set used as the test sample set.

As another embodiment, the method for base calling provided by the examples of the present application can be used for identifying the base corresponding to the designated cycle of base extension reaction and reducing the sequencing error rate. This embodiment will be hereinafter referred to as "Embodiment I".

Specifically, as shown in FIG. 2, the method for base calling provided in Embodiment I includes:
S111, determining a first sequencing information based on intensity features of a first spot in images corresponding to a consecutive cycles of base extension reactions including a designated cycle of base extension reaction.

In this step, the first sequencing information data are still derived from the intensity features of the first spot in the images corresponding to the a consecutive cycles of base extension reactions, and the a consecutive cycles of base extension reactions include the designated cycle of base extension reaction requiring base calling and the n preceding cycles of base extension reactions. In Embodiment I, the intensity features of the first spot in the images corresponding to the n completed cycles of base extension reactions and the intensity feature of the first spot in the image corresponding to the ongoing base extension reaction (the designated cycle of base extension reaction) are used as the data sources of the first sequencing information, and the base type of the ongoing base extension reaction is directly output by calling the first sequencing information formed with these data or through a preset basecall model based on the first sequencing information formed with these data. This embodiment utilizes the intensity features of the spots in the images including the designated cycle of base extension reaction, and directly outputs a base image corresponding to the designated cycle of base extension reaction based on the acquired intensity features and an established model, without identifying the bases by comparing and analyzing the brightness intensities in the four base channels. In this way, base calling can be performed for each cycle of base extension reaction one by one. Compared with the method by comparing and analyzing the brightness intensities in the four base channels, the method of Embodiment I features ease to operate, and due to the abundant sources of the first sequencing information, more factors of base calling are considered, which is favorable for improving the accuracy of base calling and reducing the sequencing error rate.

In some examples, the intensity feature is an initial intensity. The initial intensities of the first spot in the images corresponding to the a consecutive cycles of base extension reactions can be confirmed by the above method for confirming the initial intensity, which is not recited herein for brevity.

In some examples, the intensity feature is a corrected intensity. Illustratively, the corrected intensity includes at least one of a background-corrected intensity, a crosstalk-corrected intensity, and a reaction asynchrony-corrected intensity. Similarly, the corrected intensities of the first spot in the images corresponding to the a consecutive cycles of base extension reactions can be confirmed by the above method for confirming the corrected intensity, which is not recited herein for brevity.

In some examples, the information includes at least one of the following features or a combination thereof, or a feature formed by dimensionality reduction for a combination of more than one of the following features:
the ratio of an intensity corresponding to a base channel with the maximum brightness to the total intensity in the four base channels at the coordinate position corresponding to the first spot in the image acquired in the designated cycle of base extension reaction;
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} preceding cycle to the n^{th} preceding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} preceding cycle to the n^{th} preceding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} preceding cycle to the n^{th} preceding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} preceding cycle to the n^{th} preceding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

In this case, the designated cycle of base extension reaction is the ongoing base extension reaction.

The number of the n preceding cycles of base extension reactions can be flexibly selected, as long as n is a natural number greater than or equal to 1. Generally, more abundant data sources may provide a higher accuracy of base calling but reduce the efficiency of base calling due to the increased data volume. In some examples, the n preceding cycles of base extension reactions may be 1 to 5 preceding cycles of base extension reactions, that is, n is less than or equal to 5. In this case, the data sources of the first sequencing information increase, and the computation complexity caused by the increase in the data volume is relatively controllable. Illustratively, n is 1, 2, or 3, where the obtained first sequencing information may be abundant, and the first sequencing information obtained on this basis can effectively improve the accuracy of base calling.

S121, determining the base type of the designated cycle of base extension reaction based on the first sequencing information and the basecall model.

In this step, the basecall model is determined based on a second sequencing information corresponding to the a consecutive cycles of base extension reactions in a training sample and base type of at least one cycle of base extension reaction in the a consecutive cycles of base extension reactions, where the second sequencing information includes the first sequencing information.

In some examples, determining the base type of the designated cycle based on the first sequencing information and the basecall model, includes:
(1) determining the basecall model based on the number of consecutive bases of the a consecutive cycles of base extension reactions.

In this step, a matching basecall model is selected based on the number of consecutive bases in the a consecutive cycles of base extension reactions. That is, the feature value used for training the sample in the basecall model is a feature value extracted based on a base combination with the number of consecutive bases being a. Illustratively, when the a in the a consecutive cycles of base extension reactions is 3 (including the designated cycle of base extension reaction and the two preceding cycles of base extension reactions), the selected basecall model is a basecall model obtained based on training with feature values corresponding to the three base types and the three cycles of base extension reactions. Correspondingly, the feature values for training the sample in the basecall model are the intensity features of the first spot in the images corresponding to the three consecutive cycles of base extension reactions.

In one example, the base combination corresponding to the a consecutive cycles of base extension reactions is a double-base combination including a base of the designated cycle of base extension reaction and a base of the preceding cycle of base extension reaction. In this case, the first sequencing information includes at least: the ratio of an intensity corresponding to a base channel with the maximum brightness to the total intensity in the four base channels at the coordinate position corresponding to a valid spot in the image acquired in the designated cycle of base extension reaction.

(2) inputting the first sequencing information into the basecall model, outputting the base combination, and determining the base combination as the base combination corresponding to the a consecutive cycles of base extension reactions.

In this step, after the first sequencing information is input into the basecall model, the basecall model will output the base combination, and in this case, the base combination is considered as the base combination corresponding to the a consecutive cycles of base extension reactions. Illustratively, when the first sequencing information is derived from the intensity features of the first spot in the images corresponding to the 3 consecutive cycles of base extension reactions (including the designated cycle of base extension reaction and two preceding cycles of base extension reactions), inputting the first sequencing information into the basecall model will result in a three-base combination with definite base types. Illustratively, the three-base combination is GAT. In this case, the three-base combination GAT is determined as the base combination corresponding to the 3 consecutive cycles of base extension reactions.

(3) determining the base type of the designated cycle of base extension reaction based on the base combination.

In this step, with a given base combination corresponding to the a consecutive cycles of base extension reactions, the base type of the designated cycle of base extension reaction can be determined based on the sequence of the base combination.

The method for base calling provided by Embodiment I, by inputting the first sequencing feature corresponding to the designated cycle of base extension reaction on the basis of the preset basecall model, can directly output the base combination corresponding to the base number of the a consecutive cycles of base extension reactions without identifying the bases by comparing and analyzing the brightness intensities in the four base channels, so as to determine the base type of the ongoing base extension reaction. The method can achieve continuous base calling in the cycles of base extension reactions during the sequencing, thus possessing ease to operate, improved accuracy of base calling, and reduced sequencing error rate.

As one embodiment, the method for base calling provided by the examples of the present application can be used for correcting base types acquired by other base calling devices and reducing the sequencing error rate. This embodiment will be hereinafter referred to as "Embodiment II".

Specifically, as shown in FIG. 3, the method for base calling provided in Embodiment II includes:
S112, determining a first sequencing information based on intensity features of a first spot in images corresponding to a consecutive cycles of base extension reactions including a designated cycle of base extension reaction.

In this step, the source of the first sequencing information data still includes the intensity features of the first spot in the images corresponding to the a consecutive cycles of base extension reactions.

In one embodiment, the a consecutive cycles of base extension reactions include the designated cycle of base extension reaction requiring base calling and the m succeeding cycles of base extension reactions. In this case, the first sequencing information is determined by using the intensity feature of the first spot in the image corresponding to the designated cycle of base extension reaction that is completed and the intensity features of the first spot in the images corresponding to the m cycles of base extension reactions completed after the designated cycle of base extension reaction. A base combination including m + 1 bases is output based on the first sequencing information and the selected basecall model. The base identified in the designated cycle of base extension reaction is corrected via the base combination. The method, by adopting the intensity features of the first spot in the images corresponding to the m cycles of base extension reactions completed after the designated cycle of base extension reaction and the intensity feature of the first spot in the image corresponding to the designated base extension reaction (the designated cycle of base extension reaction) as the data sources of the first sequencing information, increases features influencing the base calling accuracy in the designated cycle of base extension reaction, improves the base calling accuracy, and reduces the sequencing error rate.

In some examples, the intensity feature is an initial intensity. The initial intensities of the first spot in the images corresponding to the a consecutive cycles of base extension reactions can be confirmed by the above method for confirming the initial intensity, which is not recited herein for brevity.

In some examples, the intensity feature is a corrected intensity. Illustratively, the corrected intensity includes at least one of a background-corrected intensity, a crosstalk-corrected intensity, and a reaction asynchrony-corrected intensity. Similarly, the corrected intensities of the first spot in the images corresponding to the a consecutive cycles of base extension reactions can be confirmed by the above method for confirming the corrected intensity, which is not recited herein for brevity.

In some examples of this embodiment, the first sequencing information includes at least one of the following features or a combination thereof, or a feature formed by dimensionality reduction for a combination of more than one of the following features:
the ratio of an intensity corresponding to a base channel with the maximum brightness to the total intensity in the four base channels at the coordinate position corresponding to the first spot in the image acquired in the designated cycle of base extension reaction;
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} succeeding cycle to the m^{th} succeeding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} succeeding cycle to the m^{th} succeeding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} succeeding cycle to the m^{th} succeeding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} succeeding cycle to the m^{th} succeeding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

In one example, the first sequencing information includes or is a feature formed by dimensionality reduction for the features described above.

In this embodiment, the number of the m succeeding cycles of base extension reactions can be flexibly selected, as long as m is a natural number greater than or equal to 1. Generally, a greater value of m may provide more abundant data sources and a higher accuracy of base calling, but reduce the efficiency of base calling due to the increased data volume. In some examples, the m succeeding cycles of base extension reactions may be 1 to 5 succeeding cycles of base extension reactions, i.e., m is less than or equal to 5. In this case, the data sources of the first sequencing information increase, and the computation complexity caused by the increase in the data volume is relatively controllable. Illustratively, m is 1, 2, or 3, where the obtained first sequencing information may be abundant, and the first sequencing information obtained on this basis can effectively improve the accuracy of base calling.

In another embodiment, the a consecutive cycles of base extension reactions include the designated cycle of base extension reaction requiring base calling, the n preceding cycles of base extension reactions, and the m succeeding cycles of base extension reactions. In this case, the first sequencing information is determined by using the intensity feature of the first spot in the image corresponding to the designated cycle of base extension reaction that is completed, the intensity features of the first spot in the images corresponding to the n cycles of base extension reactions completed before the designated cycle of base extension reaction, and the intensity features of the first spot in the images corresponding to the m cycles of base extension reactions completed after the designated cycle of base extension reaction. A base combination including m + n + 1 bases is output based on the first sequencing information and the selected basecall model. The base identified in the designated cycle of base extension reaction is corrected via the base combination. The method, by adopting the intensity features of the first spot in the images corresponding to the n cycles of base extension reactions completed before the designated cycle of base extension reaction, the intensity features of the first spot in the images corresponding to the m cycles of base extension reactions completed after the designated cycle of base extension reaction, and the intensity feature of the first spot in the image corresponding to the designated base extension reaction (the designated cycle of base extension reaction) as the data sources of the first sequencing information, increases features influencing the base calling accuracy in the designated cycle of base extension reaction, improves the base calling accuracy, and reduces the sequencing error rate.

In some examples of this embodiment, the information includes at least one of the following features or a combination thereof, or a feature formed by dimensionality reduction for a combination of more than one of the following features:
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} preceding cycle to the n^{th} preceding cycle of base extension reactions;
the intensity feature of the A base channel at the coordinate position corresponding to the first spot in the image acquired in each cycle of base extension reaction from the 1^{st} succeeding cycle to the m^{th} succeeding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} preceding cycle to the n^{th} preceding cycle of base extension reactions;
the intensity feature of the C base channel at the coordinate position corresponding to the first spot in the image acquired in each cycle of base extension reaction from the 1^{st} succeeding cycle to the m^{th} succeeding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} preceding cycle to the n^{th} preceding cycle of base extension reactions;
the intensity feature of the G base channel at the coordinate position corresponding to the first spot in the image acquired in each cycle of base extension reaction from the 1^{st} succeeding cycle to the m^{th} succeeding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} preceding cycle to the n^{th} preceding cycle of base extension reactions;
the intensity feature of the T/U base channel at the coordinate position corresponding to the first spot in the image acquired in each cycle of base extension reaction from the 1^{st} succeeding cycle to the m^{th} succeeding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

In this embodiment, the number of the n preceding cycles of base extension reactions can be flexibly selected, as long as n is a natural number greater than or equal to 1. Generally, more abundant data sources may provide a higher accuracy of base calling but reduce the efficiency of base calling due to the increased data volume. In some examples, the n preceding cycles of base extension reactions may be 1 to 5 preceding cycles of base extension reactions, that is, n is less than or equal to 5. In this case, the data sources of the first sequencing information increase, and the computation complexity caused by the increase in the data volume is relatively controllable. Illustratively, n is 1, 2, or 3, where the obtained first sequencing information may be abundant, and the first sequencing information obtained on this basis can effectively improve the accuracy of base calling.

The number of the m succeeding cycles of base extension reactions can be flexibly selected, as long as m is a natural number greater than or equal to 1. Generally, a greater value of m may provide more abundant data sources and a higher accuracy of base calling, but reduce the efficiency of base calling due to the increased data volume. In some examples, the m succeeding cycles of base extension reactions may be 1 to 5 succeeding cycles of base extension reactions, i.e., m is less than or equal to 5. In this case, the data sources of the first sequencing information increase, and the computation complexity caused by the increase in the data volume is relatively controllable. Illustratively, m is 1, 2, or 3, where the obtained first sequencing information may be abundant, and the first sequencing information obtained on this basis can effectively improve the accuracy of base calling.

In the two embodiments described above of this step, the base type corresponding to the a consecutive cycles of base extension reactions may be subjected to initial calling via other base calling methods.

In one example, the method includes, before the first sequencing information is determined based on the intensity features of the first spot in the images corresponding to the a consecutive cycles of base extension reactions including the designated cycle of base extension reaction, a step of subjecting the base type corresponding to the designated cycle of base extension reaction to initial calling. The step includes: mapping the coordinates of the second spots in the spot set of the sequencing template to the image corresponding to the designated cycle of base extension reaction, so as to determine the first spot in the image; correcting, based on the initial intensity of the first spot in the image, to give the corrected intensity; and determining the base type with the maximum brightness intensity in the four types of bases in the first spot based on the corrected intensity, and determining the base type as a predicted base type of the designated cycle of base extension reaction. In this case, the predicted base type of the designated cycle of base extension reaction can be corrected.

In another example, the method includes, before the first sequencing information is determined based on the intensity features of the first spot in the images corresponding to the a consecutive cycles of base extension reactions including the designated cycle of base extension reaction: mapping the coordinates of the second spots in the spot set of the sequencing template to each of the images corresponding to the a consecutive cycles of base extension reactions, so as to determine the first spot in each of the images; correcting, based on the initial intensity of the first spot in each of the images, to give the corrected intensity; determining the base type with the maximum brightness intensity in the four types of bases in the first spot based on the corrected intensity, and determining the base type as the predicted base type corresponding to the first spot; and determining the predicted combined base type based on the predicted bases corresponding to the designated cycle of base extension reaction, the n preceding cycles of base extension reactions, and the m succeeding cycles of base extension reactions. In this case, the predicted base types of the a consecutive cycles of base extension reactions including the designated cycle of base extension reaction can be corrected synchronously.

S122, determining the base type of the designated cycle of base extension reaction based on the first sequencing information and the basecall model.

In this step, the basecall model is determined based on a second sequencing information corresponding to the a consecutive cycles of base extension reactions in a training sample and base type of at least one cycle of base extension reaction in the a consecutive cycles of base extension reactions, where the second sequencing information includes the first sequencing information.

Through the preset basecall model, the base type of the designated cycle of base extension reaction can be output. This embodiment utilizes the intensity features of the spots in the images including the designated cycle of base extension reaction, and corrects the base identified in the designated cycle of base extension reaction that is completed based on the acquired intensity features and the established basecall model. In this method, the introduction of features related to base calling introduces more sources of the first sequencing information and thus more factors in base calling. As a result, the correction of the base identified in the designated cycle of base extension reaction through this method is favorable for reducing the sequencing error rate.

In some examples, determining the base type of the designated cycle based on the first sequencing information and the basecall model, includes:
(1) determining the basecall model based on the number of consecutive bases of the a consecutive cycles of base extension reactions.

In this step, a matching basecall model is selected based on the number of consecutive bases in the a consecutive cycles of base extension reactions. That is, the feature value used for training the sample in the basecall model is a feature value extracted based on a base combination with the number of consecutive bases being a. Illustratively, when the a in the a consecutive cycles of base extension reactions is 3 (including the designated cycle of base extension reaction and the two succeeding cycles of base extension reactions), the selected basecall model is a basecall model obtained based on training with feature values corresponding to the three base types and the three cycles of base extension reactions. In this case, the set target value for training the basecall model is the base type determined in the first cycle of base extension reaction (corresponding to the designated cycle of base extension reaction in the a consecutive cycles of base extension reactions) among the three bases. Correspondingly, the feature values for training the sample in the basecall model are the intensity features of the first spot in the images corresponding to the three consecutive cycles of base extension reactions.

(2) inputting the first sequencing information into the basecall model, outputting the base combination, and determining the base combination as the base combination corresponding to the a consecutive cycles of base extension reactions.

In this step, after the first sequencing information is input into the basecall model, the basecall model will output the base combination, and in this case, the base combination is considered as the base combination corresponding to the a consecutive cycles of base extension reactions. Illustratively, when the first sequencing information is derived from the intensity features of the first spot in the images corresponding to the 3 consecutive cycles of base extension reactions (including the designated cycle of base extension reaction and two succeeding cycles of base extension reactions), inputting the first sequencing information into the basecall model will result in a three-base combination with definite base types. Illustratively, the three-base combination is GAT. In this case, the three-base combination GAT is determined as the base combination corresponding to the 3 consecutive cycles of base extension reactions.

(3) correcting the predicted base type based on the base combination to determine the base type of the designated cycle of base extension reaction.

In some examples, after determining the base combination corresponding to the a consecutive cycles of base extension reactions, the base type corresponding to the designated cycle of base extension reaction in the base combination is determined by aligning the base types of the base combination with the base types corresponding to the a consecutive cycles of base extension reactions based on the sequence of the base combination. The base type is aligned with the base type obtained by initial calling in the designated cycle of base extension reaction. If the two differ, the base type output by the basecall model is used as the base type of the designated cycle of base extension reaction.

In some examples, the predicted base types corresponding to the a consecutive cycles of base extension reactions are corrected based on the base combination. Specifically, the predicted combined base types are corrected based on the base combination, so as to determine the base combination type as the base types of the a consecutive cycles of base extension reactions. That is, the base combination output by the basecall model is used as the base combination corresponding to the a consecutive cycles of base extension reactions.

In one example, an example of the method for base calling is provided.

Specifically, the method for base calling (hereinafter referred to as "two-base machine learning model") includes the following steps,
(1) performing training based on the Bayesian algorithm by using the sequencing data of a human genome library as a training set, the intensity features obtained from the sequencing reactions of the training set as the feature values, and the reference sequence base aligned to each base (i.e., the correct sequencing answers) as the target value, so as to give the basecall model. The intensity features include: the intensity feature of the A base channel at the coordinate position corresponding to the first spot in the image acquired in the designated cycle of base extension reaction; the intensity feature of the A base channel at the coordinate position corresponding to the first spot in the image acquired in each cycle of base extension reaction from the 1^{st} succeeding cycle to the m^{th} succeeding cycle of base extension reactions; the intensity feature of the C base channel at the coordinate position corresponding to the first spot in the image acquired in the designated cycle of base extension reaction; the intensity feature of the C base channel at the coordinate position corresponding to the first spot in the image acquired in each cycle of base extension reaction from the 1^{st} succeeding cycle to the m^{th} succeeding cycle of base extension reactions; the intensity feature of the G base channel at the coordinate position corresponding to the first spot in the image acquired in the designated cycle of base extension reaction; the intensity feature of the G base channel at the coordinate position corresponding to the first spot in the image acquired in each cycle of base extension reaction from the 1^{st} succeeding cycle to the m^{th} succeeding cycle of base extension reactions; the intensity feature of the T/U base channel at the coordinate position corresponding to the first spot in the image acquired in the designated cycle of base extension reaction; and the intensity feature of the T/U base channel at the coordinate position corresponding to the first spot in the image acquired in each cycle of base extension reaction from the 1^{st} succeeding cycle to the m^{th} succeeding cycle of base extension reactions.
(2) determining a first sequencing information based on intensity features of a first spot in images corresponding to 2 consecutive cycles of base extension reactions including a designated cycle of base extension reaction and a succeeding cycle of base extension reaction on the basis of the human genome library. The first sequencing information includes: the intensity feature of the A base channel at the coordinate position corresponding to the first spot in the image acquired in the designated cycle of base extension reaction; the intensity feature of the A base channel at the coordinate position corresponding to the first spot in the image acquired in each cycle of base extension reaction from the 1^{st} succeeding cycle to the m^{th} succeeding cycle of base extension reactions; the intensity feature of the C base channel at the coordinate position corresponding to the first spot in the image acquired in the designated cycle of base extension reaction; the intensity feature of the C base channel at the coordinate position corresponding to the first spot in the image acquired in each cycle of base extension reaction from the 1^{st} succeeding cycle to the m^{th} succeeding cycle of base extension reactions; the intensity feature of the G base channel at the coordinate position corresponding to the first spot in the image acquired in the designated cycle of base extension reaction; the intensity feature of the G base channel at the coordinate position corresponding to the first spot in the image acquired in each cycle of base extension reaction from the 1^{st} succeeding cycle to the m^{th} succeeding cycle of base extension reactions; the intensity feature of the T/U base channel at the coordinate position corresponding to the first spot in the image acquired in the designated cycle of base extension reaction; and the intensity feature of the T/U base channel at the coordinate position corresponding to the first spot in the image acquired in each cycle of base extension reaction from the 1^{st} succeeding cycle to the m^{th} succeeding cycle of base extension reactions.
(3) performing sequencing on the human genome library and an *Escherichia coli* library as the validation sets by using the basecall model described above and reading the sequencing data.

The data obtained from directly sequencing the human genome library and the *E. coli* library without using the basecall model are used as the reference (hereinafter referred to as "original base calling algorithm").

The base type of the designated cycle of base extension reaction is determined based on the first sequencing information and the basecall model, where the basecall model is determined based on a second sequencing information corresponding to the a consecutive cycles of base extension reactions in a training sample and base type of at least one cycle of base extension reaction in the a consecutive cycles of base extension reactions, and the second sequencing information includes the first sequencing information.

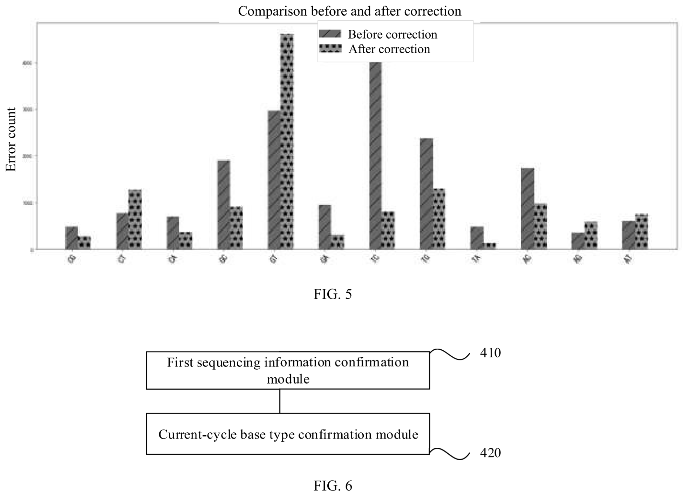

The image of the selected field of view in the original sequencing image was randomly selected for analysis. The error counts corresponding to the original base calling algorithm used for comparison and the two-base machine learning model were separately determined, along with the error correction rate of the machine learning model. Specifically, Table 1 shows the comparison of the error counts by error types and the error correction statistics obtained by sequencing the human genome sample using the original base calling algorithm (Algorithm 1) and the two-base machine learning model (Algorithm 2). Table 2 shows the comparison of the error counts by error types and the error correction statistics obtained by sequencing the *E*. *coli* sample using the original base calling algorithm (Algorithm 1) and the two-base machine learning model (Algorithm 2). Correspondingly, FIG. 4 shows the comparison of error counts by error types for sequencing the human genome sample using the two-base machine learning model and the original base calling algorithm according to one example; and FIG. 5 shows the comparison of error counts by error types for sequencing the *E. coli* sample using the two-base machine learning model and the original base calling algorithm according to one example. CG denotes that base C was mistakenly identified as base G. Other error types shall be interpreted similarly. For example, CT denotes that base C was mistakenly identified as base T. For brevity, the details are not recited here.

**Table 1**

| Error type | CG | CT | CA | GC | GT | GA | TC | TG | TA | AC | AG | AT | Total error count |
|---|---|---|---|---|---|---|---|---|---|---|---|---|---|
| Error count of Algorithm 1 | 14086 | 16213 | 8571 | 5593 | 8167 | 8331 | 10538 | 11668 | 8334 | 5560 | 9803 | 3371 | 110235 |
| Error count of Algorithm 2 | 4089 | 7392 | 3746 | 2462 | 4465 | 3976 | 4649 | 3813 | 2017 | 3418 | 5211 | 2892 | 48130 |
| Error correction rate | 0.710 | 0.544 | 0.563 | 0.560 | 0.453 | 0.523 | 0.559 | 0.673 | 0.758 | 0.385 | 0.468 | 0.142 | 0.563 |

**Table 2**

| Error type | AC | AG | AT | CA | CG | CT | GA | GC | GT | TA | TC | TG | Total error count |
|---|---|---|---|---|---|---|---|---|---|---|---|---|---|
| Error count of Algorithm 1 | 480 | 774 | 702 | 1901 | 2966 | 947 | 4024 | 2376 | 480 | 1737 | 358 | 598 | 17343 |
| Error count of Algorithm 2 | 280 | 1273 | 371 | 897 | 4612 | 314 | 807 | 1288 | 127 | 978 | 588 | 743 | 12278 |
| Error correction rate | 0.417 | -0.645 | 0.472 | 0.528 | -0.555 | 0.668 | 0.799 | 0.458 | 0.735 | 0.437 | -0.642 | -0.242 | 0.292 |

As shown in the figures and tables, after applying the machine learning algorithm provided in the example of the present application, the overall sequencing error rate was reduced by about 25% to 30%.

In another example of the present application, an apparatus for base calling is provided. The apparatus provided in the example of the present application is capable of executing the methods for base calling provided in any of the examples of the present application, and includes the corresponding functional modules and achieves the associated beneficial effects.

FIG. 6 illustrates a schematic of the apparatus for base calling provided in the example of the present application. As shown in FIG. 6, the apparatus includes: a first sequencing information confirmation module 410 and a designated-cycle base type confirmation module 420, where:
the first sequencing information confirmation module 410 is configured for determining a first sequencing information based on intensity features of a first spot in images corresponding to a consecutive cycles of base extension reactions including a designated cycle of base extension reaction, where a is a natural number greater than or equal to 1;
the designated-cycle base type confirmation module 420 is configured for determining a base type of the designated cycle of base extension reaction based on the first sequencing information and a basecall model, where the basecall model is determined based on a second sequencing information corresponding to the a consecutive cycles of base extension reactions in a training sample and base type of at least one cycle of base extension reaction in the a consecutive cycles of base extension reactions, and the second sequencing information includes the first sequencing information.

Further, in the examples of the present application, the a consecutive cycles of base extension reactions include a designated cycle of base extension reaction requiring base calling, and at least one of the n preceding cycles of base extension reactions and the m succeeding cycles of base extension reactions, where n is an integer greater than or equal to 0, and m is an integer greater than or equal to 0.

Further, in the examples of the present application, the first sequencing information includes at least one of the following features or a combination thereof, or a feature formed by dimensionality reduction for a combination of more than one of the following features: the ratio of an intensity corresponding to a base channel with the maximum brightness to the total intensity in the four base channels at the coordinate position corresponding to the first spot in the image acquired in the designated cycle of base extension reaction; the total number of the first spots in the image acquired in the designated cycle of base extension reaction; the intensity feature of the A base channel at the coordinate position corresponding to the first spot in the image acquired in the designated cycle of base extension reaction; the intensity feature of the A base channel at the coordinate position corresponding to the first spot in the image acquired in each cycle of base extension reaction from the 1^{st} preceding cycle to the n^{th} preceding cycle of base extension reactions; the intensity feature of the A base channel at the coordinate position corresponding to the first spot in the image acquired in each cycle of base extension reaction from the 1^{st} succeeding cycle to the m^{th} succeeding cycle of base extension reactions; the intensity feature of the C base channel at the coordinate position corresponding to the first spot in the image acquired in the designated cycle of base extension reaction; the intensity feature of the C base channel at the coordinate position corresponding to the first spot in the image acquired in each cycle of base extension reaction from the 1^{st} preceding cycle to the n^{th} preceding cycle of base extension reactions; the intensity feature of the C base channel at the coordinate position corresponding to the first spot in the image acquired in each cycle of base extension reaction from the 1^{st} succeeding cycle to the m^{th} succeeding cycle of base extension reactions; the intensity feature of the G base channel at the coordinate position corresponding to the first spot in the image acquired in the designated cycle of base extension reaction; the intensity feature of the G base channel at the coordinate position corresponding to the first spot in the image acquired in each cycle of base extension reaction from the 1^{st} preceding cycle to the n^{th} preceding cycle of base extension reactions; the intensity feature of the G base channel at the coordinate position corresponding to the first spot in the image acquired in each cycle of base extension reaction from the 1^{st} succeeding cycle to the m^{th} succeeding cycle of base extension reactions; the intensity feature of the T/U base channel at the coordinate position corresponding to the first spot in the image acquired in the designated cycle of base extension reaction; the intensity feature of the T/U base channel at the coordinate position corresponding to the first spot in the image acquired in each cycle of base extension reaction from the 1^{st} preceding cycle to the n^{th} preceding cycle of base extension reactions; the intensity feature of the T/U base channel at the coordinate position corresponding to the first spot in the image acquired in each cycle of base extension reaction from the 1^{st} succeeding cycle to the m^{th} succeeding cycle of base extension reactions; the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

Further, in the examples of the present application, the apparatus further includes: an intensity feature determination module, configured for mapping the coordinates of second spots in a spot set of a sequencing template to each of the images, so as to determine the first spot in the images and determining the intensity features of the first spot in the images corresponding to the a consecutive cycles of base extension reactions based on the first spot in each of the images.

Further, in the examples of the present application, the intensity feature determination module is further configured for: mapping the coordinates of the second spots in the spot set of the sequencing template to each of the images, so as to determine a position corresponding to the coordinates of the second spot in the image, and determining a spot corresponding to the position as the first spot.

Further, in the examples of the present application, the intensity feature includes any one of an initial intensity and a corrected intensity.

Further, in the examples of the present application, the intensity feature includes the initial intensity, and the module for confirming the intensity features of the first spot in the images corresponding to the a consecutive cycles of base extension reactions is configured for: acquiring the images obtained from the a consecutive cycles of base extension reactions, extracting original intensities of the A base channel, the C base channel, the G base channel, and the T/U base channel at the first spot in the images, and confirming that the original intensities of the A base channel, the C base channel, the G base channel, and the T/U base channel are the initial intensities of the A base channel, the C base channel, the G base channel, and the T/U base channel at the first spot, respectively.

Further, in the examples of the present application, the corrected intensity includes at least one of a background-corrected intensity, a crosstalk-corrected intensity, and a reaction asynchrony-corrected intensity.

Further, in the examples of the present application, the module for confirming the intensity features of the first spot in the images corresponding to the a consecutive cycles of base extension reactions is further configured for: acquiring the images obtained from the a consecutive cycles of base extension reactions, extracting original intensities of the A base channel, the C base channel, the G base channel, and the T/U base channel at the first spot in the images, and confirming that the original intensities of the A base channel, the C base channel, the G base channel, and the T/U base channel are the initial intensities of the A base channel, the C base channel, the G base channel, and the T/U base channel at the first spot, respectively; at least one of the background correction, crosstalk correction, and reaction asynchrony correction is performed based on the initial intensities to confirm the corrected intensity.

Further, in the examples of the present application, the designated-cycle base type confirmation module is further configured for: determining a base combination type corresponding to the a consecutive cycles of base extension reactions; and inputting the first sequencing information into the basecall model matching the base combination type, outputting a base combination corresponding to the a consecutive cycles of base extension reactions, and determining the base type of the designated cycle of base extension reaction based on the base combination.

Further, in the examples of the present application, the module for establishing the basecall model is configured for: acquiring a training sample set, where each sample in the training sample set is labeled with a feature value and a target value, the feature value is a second sequencing information determined based on the base combination type, and the target value is the base type corresponding to the designated cycle of base extension reaction in the base combination type; and performing machine learning modeling on each sample in the training sample set based on a specific model structure to give the basecall model.

Further, in the examples of the present application, the specific model structure is the lightGMB model.

Further, in the examples of the present application, the a consecutive cycles of base extension reactions include the designated cycle of base extension reaction requiring base calling and the n preceding cycles of base extension reactions, where n is a natural number greater than or equal to 1.

Further, in the examples of the present application, n is less than or equal to 5.

Further, in the examples of the present application, n is 1, 2, or 3.

Further, in the examples of the present application, the first sequencing information includes at least one of the following features or a combination thereof, or a feature formed by dimensionality reduction for a combination of more than one of the following features: the ratio of an intensity corresponding to a base channel with the maximum brightness to the total intensity in the four base channels at the coordinate position corresponding to the first spot in the image acquired in the designated cycle of base extension reaction; the total number of the first spots in the image acquired in the designated cycle of base extension reaction; the intensity feature of the A base channel at the coordinate position corresponding to the first spot in the image acquired in the designated cycle of base extension reaction; the intensity feature of the A base channel at the coordinate position corresponding to the first spot in the image acquired in each cycle of base extension reaction from the 1^{st} preceding cycle to the n^{th} preceding cycle of base extension reactions; the intensity feature of the C base channel at the coordinate position corresponding to the first spot in the image acquired in the designated cycle of base extension reaction; the intensity feature of the C base channel at the coordinate position corresponding to the first spot in the image acquired in each cycle of base extension reaction from the 1^{st} preceding cycle to the n^{th} preceding cycle of base extension reactions; the intensity feature of the G base channel at the coordinate position corresponding to the first spot in the image acquired in the designated cycle of base extension reaction; the intensity feature of the G base channel at the coordinate position corresponding to the first spot in the image acquired in each cycle of base extension reaction from the 1^{st} preceding cycle to the n^{th} preceding cycle of base extension reactions; the intensity feature of the T/U base channel at the coordinate position corresponding to the first spot in the image acquired in the designated cycle of base extension reaction; the intensity feature of the T/U base channel at the coordinate position corresponding to the first spot in the image acquired in each cycle of base extension reaction from the 1^{st} preceding cycle to the n^{th} preceding cycle of base extension reactions; the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and the standard deviation of the intensities corresponding to the four base channels at the coordinate position corresponding to the first spot in the image acquired in the designated cycle of base extension reaction.

Further, in the examples of the present application, the designated-cycle base type confirmation module is further configured for: determining the basecall model based on the number of consecutive bases of the a consecutive cycles of base extension reactions; inputting the first sequencing information into the basecall model, outputting the base combination, and determining the base combination as the base combination corresponding to the a consecutive cycles of base extension reactions; and determining the base type of the designated cycle of base extension reaction based on the base combination.

Further, in the examples of the present application, the base combination corresponding to the a consecutive cycles of base extension reactions is a double-base combination including a base of the designated cycle of base extension reaction and a base of the preceding cycle of base extension reaction.

Further, in the examples of the present application, the first sequencing information includes: the ratio of an intensity corresponding to a base channel with the maximum brightness to the total intensity in the four base channels at the coordinate position corresponding to a valid spot in the image acquired in the designated cycle of base extension reaction.

Further, in the examples of the present application, the a consecutive cycles of base extension reactions include the designated cycle of base extension reaction requiring base calling and the m succeeding cycles of base extension reactions, where m is a natural number greater than or equal to 1.

Further, in the examples of the present application, m is less than or equal to 5.

Further, in the examples of the present application, m is 1, 2, or 3.

Further, in the examples of the present application, the first sequencing information includes at least one of the following features or a combination thereof, or a feature formed by dimensionality reduction for a combination of more than one of the following features: the ratio of an intensity corresponding to a base channel with the maximum brightness to the total intensity in the four base channels at the coordinate position corresponding to the first spot in the image acquired in the designated cycle of base extension reaction; the total number of the first spots in the image acquired in the designated cycle of base extension reaction; the intensity feature of the A base channel at the coordinate position corresponding to the first spot in the image acquired in the designated cycle of base extension reaction; the intensity feature of the A base channel at the coordinate position corresponding to the first spot in the image acquired in each cycle of base extension reaction from the 1^{st} succeeding cycle to the m^{th} succeeding cycle of base extension reactions; the intensity feature of the C base channel at the coordinate position corresponding to the first spot in the image acquired in the designated cycle of base extension reaction; the intensity feature of the C base channel at the coordinate position corresponding to the first spot in the image acquired in each cycle of base extension reaction from the 1^{st} succeeding cycle to the m^{th} succeeding cycle of base extension reactions; the intensity feature of the G base channel at the coordinate position corresponding to the first spot in the image acquired in the designated cycle of base extension reaction; the intensity feature of the G base channel at the coordinate position corresponding to the first spot in the image acquired in each cycle of base extension reaction from the 1^{st} succeeding cycle to the m^{th} succeeding cycle of base extension reactions; the intensity feature of the T/U base channel at the coordinate position corresponding to the first spot in the image acquired in the designated cycle of base extension reaction; the intensity feature of the T/U base channel at the coordinate position corresponding to the first spot in the image acquired in each cycle of base extension reaction from the 1^{st} succeeding cycle to the m^{th} succeeding cycle of base extension reactions; the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and the standard deviation of the intensities corresponding to the four base channels at the coordinate position corresponding to the first spot in the image acquired in the designated cycle of base extension reaction.

Further, in the examples of the present application, the apparatus further includes: the first predicted base type, configured for respectively mapping the coordinates of the second spots in the spot set of the sequencing template to each of the images, so as to determine the first spots in the images; correcting, based on the initial intensity of the first spot in each of the images, to give the corrected intensity; and determining the base type with the maximum brightness intensity in the four types of bases in the first spot based on the corrected intensity, and determining the base type as a predicted base type of the designated cycle of base extension reaction.

Further, in the examples of the present application, the designated-cycle base type confirmation module is further configured for: determining the basecall model based on the number of the consecutive bases of the a consecutive cycles of base extension reactions; inputting the first sequencing information into the basecall model, outputting the base combination, and determining the base combination as the base combination corresponding to the a consecutive cycles of base extension reactions; and correcting the predicted base type based on the base combination to determine the base type of the designated cycle of base extension reaction.

Further, in the examples of the present application, the a consecutive cycles of base extension reactions include the designated cycle of base extension reaction, the n preceding cycles of base extension reactions, and the m succeeding cycles of base extension reactions, where n and m are both natural numbers greater than or equal to 1.

Further, in the examples of the present application, n is less than or equal to 5, and/or m is less than or equal to 5.

Further, in the examples of the present application, n is 1, 2, or 3, and/or m is 1, 2, or 3.

Further, in the examples of the present application, the apparatus further includes a second predicted base type determination module, configured for: mapping the coordinates of the second spots in the spot set of the sequencing template to the image corresponding to the designated cycle of base extension reaction, so as to determine the first spot in the image; correcting, based on the initial intensity of the first spot in the image, to give the corrected intensity; and determining the base type with the maximum brightness intensity in the four types of bases in the first spot based on the corrected intensity, and determining the base type as a predicted base type of the designated cycle of base extension reaction.

Further, in the examples of the present application, the designated-cycle base type confirmation module is further configured for: determining the basecall model based on the number of the consecutive bases of the a consecutive cycles of base extension reactions; inputting the first sequencing information into the basecall model, outputting the model base combination, and determining the model base combination as the base combination corresponding to the a consecutive cycles of base extension reactions; and correcting the predicted base type based on the base combination to determine the base type of the designated cycle of base extension reaction.

Further, in the examples of the present application, the apparatus further includes a predicted combined base type determination module, configured for: mapping the coordinates of the second spots in the spot set of the sequencing template to the images corresponding to the a consecutive cycles of base extension reactions, so as to determine the position of the first spot in each of the images; correcting, based on the initial intensity of the first spot in each of the images, to give the corrected intensity; determining the base type with the maximum brightness intensity in the four types of bases in the first spot based on the corrected intensity, and determining the base type as a predicted base type corresponding to the first spot; and determining predicted combined base types based on the predicted bases corresponding to the designated cycle of base extension reaction, the n preceding cycles of base extension reactions, and the m succeeding cycles of base extension reactions.

Further, in the examples of the present application, the designated-cycle base type confirmation module is further configured for: determining the basecall model based on the number of the consecutive bases of the a consecutive cycles of base extension reactions; inputting the first sequencing information into the basecall model, and outputting the base combination; and correcting the predicted combined base type based on the base combination to determine the base combination type as the base type of the a consecutive cycles of base extension reactions.

According to the technical schemes of the examples of the present application, the first sequencing information is determined through the intensity features of the first spot in the images corresponding to the a consecutive cycles of base extension reactions including the designated cycle of base extension reaction, and the base type of the designated cycle is obtained according to the first sequencing information and the basecall model. The first sequencing information is determined according to the intensity features of the combined bases, such that the information of the base in the designated cycle in the first sequencing information is more comprehensive, and the base type of the designated cycle can be determined according to the basecall model and the first sequencing information, thus helping more accurate identification of the types and the sequence of the bases binding to the template nucleic acid in the base extension reaction and improving the sequencing accuracy.

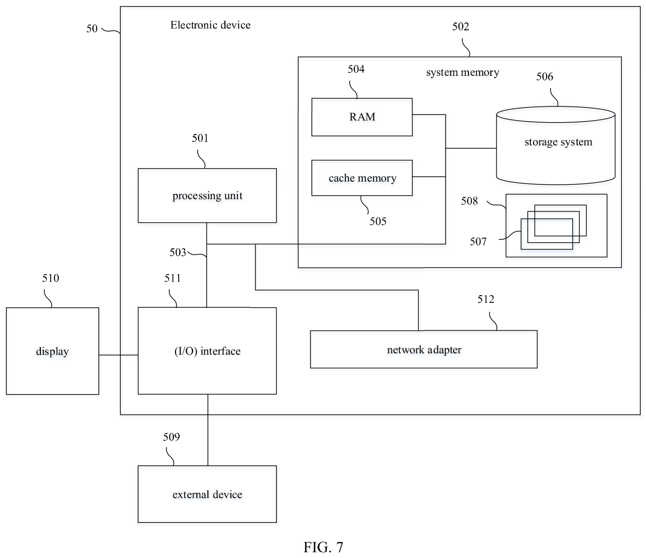

In another example of the present application, an electronic device is provided. FIG. 7 illustrates a block diagram of an exemplary electronic device 50 suitable for use in implementing the embodiments of the present application. The electronic device 50 shown in FIG. 7 is only an example, and shall not be construed as any limitation to the scope of functionality and usage in the examples of the present application.

As shown in FIG. 7, the electronic device 50 may be present in the form of a general-purpose computing device. The components of the electronic device 50 may include, but are not limited to: one or more processors or processing units 501, a system memory 502, and a bus 503 connecting various system components (including the system memory 502 and the processing unit 501).

The bus 503 represents one or more of several types of bus structures, including a memory bus or a memory controller, a peripheral bus, an accelerated graphics port, a processor, or a local area bus using any of a variety of bus structures. By way of example, such architectures include, but are not limited to, the Industry Standard Architecture (ISA) bus, the Microchannel Architecture (MCA) bus, the Enhanced ISA bus, the Video Electronics Standards Association (VESA) local area bus, and the Peripheral Component Interconnect (PCI) bus.

The electronic device 50 typically includes a variety of computer system-readable media. Such media may be any available media that is accessible by the electronic device 50, including both volatile and non-volatile media, and both removable and non-removable media.

The system memory 502 may include a computer system-readable medium in the form of a volatile memory, such as a random access memory (RAM) 504 and/or a cache memory 505. The electronic device 50 may further include other removable/non-removable, volatile/non-volatile computer system storage media. By way of example only, a storage system 506 may be used to read from and write to a non-removable, non-volatile magnetic medium (not shown in FIG. 7; commonly known as a "hard disk drive"). Although not shown in FIG. 7, a magnetic disk drive for reading from and writing to a removable, non-volatile magnetic disk (e.g., a "soft disk") and an optical disk drive for reading from or writing to a removable, non-volatile optical disk (e.g., a CD-ROM, a DVD-ROM, or other optical media) may be provided. In such cases, each drive may be connected to the bus 503 by one or more data media interfaces. The memory 502 may include at least one program product having a set of (e.g., at least one) program modules that are configured to execute the functions according to the examples of the present application.

A program/utility 508 having a set of (at least one) program modules 507 may be stored, for example, in the memory 502. Such program modules 507 include, but are not limited to: an operation system, one or more applications, other program modules, and program data. Each of the examples or a combination thereof may include the implementation of a network environment. The program modules 507 generally execute the functionality and/or the method according to the examples of the present application as described herein.

The electronic device 50 may also communicate with one or more external devices 509 (e.g., a keyboard, a pointing device, a display 510, etc.), one or more devices that enable a user to interact with the electronic device 50, and/or any device (e.g., a network card, a modem, etc.) that enables the electronic device 50 to communicate with one or more other computing devices. Such communication may be implemented through an input/output (I/O) interface 511. In addition, the electronic device 50 may also communicate with one or more networks (e.g., a local area network (LAN), a wide area network (WAN), and/or a public network, such as the Internet) via a network adapter 512. As shown, the network adapter 512 communicates with the other modules of the electronic device 50 via the bus 503. It will be appreciated that although not shown in FIG. 3, other hardware and/or software modules may be used in conjunction with the electronic device 50, including, but not limited to: microcode, device drivers, redundant processing units, external disk drive arrays, RAID systems, tape drives, data backup storage systems, and the like.

The processing unit 501 executes various functional applications and data processing, such as implementing the method for base calling provided in the examples of the present application, by running a program stored in the system memory 502.

In another example of the present application, a storage medium including a computer-executable instruction is provided, where the computer-executable instruction is configured for, when executed by a computer processor, implementing a method for base calling, including:
determining a first sequencing information based on intensity features of a first spot in images corresponding to a consecutive cycles of base extension reactions including a designated cycle of base extension reaction, where a is a natural number greater than or equal to 1; and determining a base type of the designated cycle of base extension reaction based on the first sequencing information and a basecall model, where the basecall model is determined based on a second sequencing information corresponding to the a consecutive cycles of base extension reactions in a training sample and base type of at least one cycle of base extension reaction in the a consecutive cycles of base extension reactions, and the second sequencing information includes the first sequencing information.

According to the examples of the present application, the computer storage medium may take the form of any combination of one or more computer-readable media. The computer-readable medium may be a computer-readable signal medium or a computer-readable storage medium. The computer-readable storage medium may be, but is not limited to, for example, an electronic, magnetic, optical, electromagnetic, infrared, or semiconductor system, apparatus, or device, or any combination of the foregoing. More specific examples (a non-exhaustive list) of the computer-readable storage medium include the following: an electrical connection having one or more wires, a portable computer magnetic disk, a hard disk, a random access memory (RAM), a read-only memory (ROM), an erasable programmable read-only memory (EPROM or flash memory), an optical fiber, a portable compact disc read-only memory (CD-ROM), an optical storage device, a magnetic storage device, or any suitable combination of the foregoing. In the context of this document, the computer-readable storage medium may be any tangible medium that includes or stores a program for use by or in connection with an instruction execution system, apparatus, or device.

The computer-readable signal medium may include a data signal propagated in baseband or as part of a carrier wave, where the data signal carries a computer-readable program code. Such a propagated data signal may take various forms, including, but not limited to, electromagnetic signals, optical signals, or any suitable combination of the foregoing. The computer-readable signal medium may also be any computer-readable medium that is not a computer-readable storage medium and that can send, propagate, or transmit a program for use by or in conjunction with the instruction execution system, apparatus, or device.

The program code included on the computer-readable medium may be transmitted using any appropriate medium, including, but not limited to, wireless, wireline, optical fiber cable, RF, etc., or any suitable combination of the foregoing.

The computer program code for conducting the operations according to the examples of the present application may be written in one or more programming languages, or a combination thereof. The programming languages include object-oriented programming languages-such as Java, Smalltalk, or C++-as well as conventional procedural programming languages-such as the "C" programming language or similar programming languages. The program code may be executed entirely on a user computer, partly on a user computer, as a standalone software package, partly on a user computer and partly on a remote computer, or entirely on a remote computer or server. In the case of a remote computer, the remote computer may be connected to the user computer through any type of network, including a local area network (LAN) or a wide area network (WAN), or may be connected to an external computer (for example, via the Internet using an Internet Service Provider).

The above disclosure is only for the purpose of illustrating the preferred examples of the present application and should not be construed as limiting the claimed scope of the present application. Therefore, equivalent changes made to the claims of the present application shall still fall within the scope of the present application.

## Claims

1. A method for base calling, comprising:
determining a first sequencing information based on intensity features of a first spot in images corresponding to a consecutive cycles of base extension reactions comprising a designated cycle of base extension reaction, wherein a is a natural number greater than or equal to 1; and
determining a base type of the designated cycle of base extension reaction based on the first sequencing information and a basecall model, wherein the basecall model is determined based on a second sequencing information corresponding to the a consecutive cycles of base extension reactions in a training sample and base type of at least one cycle of base extension reaction in the a consecutive cycles of base extension reactions, and the second sequencing information comprises the first sequencing information.

2. The method according to claim 1, wherein the a consecutive cycles of base extension reactions comprise a designated cycle of base extension reaction requiring base calling, and at least one of the n preceding cycles of base extension reactions and the m succeeding cycles of base extension reactions, wherein n is an integer greater than or equal to 0, and m is an integer greater than or equal to 0.

3. The method according to claim 2, wherein the first sequencing information comprises at least one of the following features or a combination thereof, or a feature formed by dimensionality reduction for a combination of more than one of the following features:
the ratio of an intensity corresponding to a base channel with the maximum brightness to the total intensity in the four base channels at the coordinate position corresponding to the first spot in the image acquired in the designated cycle of base extension reaction;
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} preceding cycle to the n^{th} preceding cycle of base extension reactions;
the intensity feature of the A base channel at the coordinate position corresponding to the first spot in the image acquired in each cycle of base extension reaction from the 1^{st} succeeding cycle to the m^{th} succeeding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} preceding cycle to the n^{th} preceding cycle of base extension reactions;
the intensity feature of the C base channel at the coordinate position corresponding to the first spot in the image acquired in each cycle of base extension reaction from the 1^{st} succeeding cycle to the m^{th} succeeding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} preceding cycle to the n^{th} preceding cycle of base extension reactions;
the intensity feature of the G base channel at the coordinate position corresponding to the first spot in the image acquired in each cycle of base extension reaction from the 1^{st} succeeding cycle to the m^{th} succeeding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} preceding cycle to the n^{th} preceding cycle of base extension reactions;
the intensity feature of the T/U base channel at the coordinate position corresponding to the first spot in the image acquired in each cycle of base extension reaction from the 1^{st} succeeding cycle to the m^{th} succeeding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

4. The method according to any one of claims 1-3, further comprising, prior to determining a first sequencing information based on intensity features of a first spot in images corresponding to a consecutive cycles of base extension reactions comprising a designated cycle of base extension reaction:
mapping the coordinates of second spots in a spot set of a sequencing template to each of the images, so as to determine the first spot in the images; and
determining the intensity features of the first spot in the images corresponding to the a consecutive cycles of base extension reactions based on the first spot in each of the images.

5. The method according to claim 4, wherein mapping the coordinates of second spots in a spot set of a sequencing template to each of the images, so as to determine the first spot in the images, comprises:
mapping the coordinates of the second spots in the spot set of the sequencing template to each of the images, so as to determine a position corresponding to the coordinates of the second spot in the image, and determining a spot corresponding to the position as the first spot.

6. The method according to any one of claims 1-3, wherein the intensity feature comprises any one of an initial intensity and a corrected intensity.

7. The method according to claim 6, wherein the intensity feature comprises the initial intensity, and the method for confirming the intensity features of the first spot in the images corresponding to the a consecutive cycles of base extension reactions comprises:
acquiring the images obtained from the a consecutive cycles of base extension reactions, extracting original intensities of the A base channel, the C base channel, the G base channel, and the T/U base channel at the first spot in the images, and confirming that the original intensities of the A base channel, the C base channel, the G base channel, and the T/U base channel are the initial intensities of the A base channel, the C base channel, the G base channel, and the T/U base channel at the first spot, respectively.

8. The method according to claim 6, wherein the corrected intensity comprises at least one of a background-corrected intensity, a crosstalk-corrected intensity, and a reaction asynchrony-corrected intensity.

9. The method according to claim 8, wherein the method for confirming the intensity features of the first spot in the images corresponding to the a consecutive cycles of base extension reactions comprises:
acquiring the images obtained from the a consecutive cycles of base extension reactions, extracting original intensities of the A base channel, the C base channel, the G base channel, and the T/U base channel at the first spot in the images, and confirming that the original intensities of the A base channel, the C base channel, the G base channel, and the T/U base channel are the initial intensities of the A base channel, the C base channel, the G base channel, and the T/U base channel at the first spot, respectively; and
confirming the corrected intensity by performing at least one of background correction, crosstalk correction, and reaction asynchrony correction based on the initial intensities.

10. The method according to any one of claims 1-9, wherein determining a base type of the designated cycle of base extension reaction based on the first sequencing information and a basecall model comprises:
determining a base combination type corresponding to the a consecutive cycles of base extension reactions;
and
inputting the first sequencing information into the basecall model matching the base combination type, outputting a base combination corresponding to the a consecutive cycles of base extension reactions, and determining the base type of the designated cycle of base extension reaction based on the base combination.

11. The method according to claim 10, wherein the method for establishing the basecall model comprises:
acquiring a training sample set, wherein each sample in the training sample set is labeled with a feature value and a target value, the feature value is a second sequencing information determined based on the base combination type, and the target value is the base type corresponding to the designated cycle of base extension reaction in the base combination type; and
performing machine learning modeling on each sample in the training sample set based on a specific model structure to give the basecall model.

12. The method according to claim 11, wherein the specific model structure is the lightGMB model.

13. The method according to any one of claims 1-12, wherein the a consecutive cycles of base extension reactions comprise the designated cycle of base extension reaction requiring base calling and the n preceding cycles of base extension reactions, wherein n is a natural number greater than or equal to 1.

14. The method according to claim 13, wherein n is less than or equal to 5.

15. The method according to claim 14, wherein n is 1, 2, or 3.

16. The method according to any one of claims 13-15, wherein the first sequencing information comprises at least one of the following features or a combination thereof, or a feature formed by dimensionality reduction for a combination of more than one of the following features:
the ratio of an intensity corresponding to a base channel with the maximum brightness to the total intensity in the four base channels at the coordinate position corresponding to the first spot in the image acquired in the designated cycle of base extension reaction;
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} preceding cycle to the n^{th} preceding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} preceding cycle to the n^{th} preceding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} preceding cycle to the n^{th} preceding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} preceding cycle to the n^{th} preceding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

17. The method according to claim 16, wherein determining a base type of the designated cycle of base extension reaction based on the first sequencing information and a basecall model comprises:
determining the basecall model based on the number of consecutive bases of the a consecutive cycles of base extension reactions;
inputting the first sequencing information into the basecall model, outputting the base combination, and determining the base combination as the base combination corresponding to the a consecutive cycles of base extension reactions; and
determining the base type of the designated cycle of base extension reaction based on the base combination.

18. The method according to any one of claims 13-17, wherein the base combination corresponding to the a consecutive cycles of base extension reactions is a double-base combination comprising a base of the designated cycle of base extension reaction and a base of the preceding cycle of base extension reaction.

19. The method according to claim 18, wherein the first sequencing information comprises: the ratio of an intensity corresponding to a base channel with the maximum brightness to the total intensity in the four base channels at the coordinate position corresponding to a valid spot in the image acquired in the designated cycle of base extension reaction.

20. The method according to any one of claims 1-12, wherein the a consecutive cycles of base extension reactions comprise the designated cycle of base extension reaction requiring base calling and the m succeeding cycles of base extension reactions, wherein m is a natural number greater than or equal to 1.

21. The method according to claim 20, wherein m is less than or equal to 5.

22. The method according to claim 21, wherein m is 1, 2, or 3.

23. The method according to any one of claims 20-22, wherein the first sequencing information comprises at least one of the following features or a combination thereof, or a feature formed by dimensionality reduction for a combination of more than one of the following features:
the ratio of an intensity corresponding to a base channel with the maximum brightness to the total intensity in the four base channels at the coordinate position corresponding to the first spot in the image acquired in the designated cycle of base extension reaction;
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} succeeding cycle to the m^{th} succeeding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} succeeding cycle to the m^{th} succeeding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} succeeding cycle to the m^{th} succeeding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} succeeding cycle to the m^{th} succeeding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

24. The method according to claim 23, comprising, prior to determining a first sequencing information based on intensity features of a first spot in images corresponding to a consecutive cycles of base extension reactions comprising a designated cycle of base extension reaction:
mapping the coordinates of the second spots in the spot set of the sequencing template to each of the images, so as to determine the first spot in the images;
correcting, based on the initial intensity of the first spot in each of the images, to give the corrected intensity;
and
determining the base type with the maximum brightness intensity in the four types of bases in the first spot based on the corrected intensity, and determining the base type as a predicted base type of the designated cycle of base extension reaction.

25. The method according to claim 24, wherein determining a base type of the designated cycle of base extension reaction based on the first sequencing information and a basecall model comprises:
determining the basecall model based on the number of consecutive bases of the a consecutive cycles of base extension reactions;
inputting the first sequencing information into the basecall model, outputting the base combination, and determining the base combination as the base combination corresponding to the a consecutive cycles of base extension reactions; and
correcting the predicted base type based on the base combination.

26. The method according to any one of claims 1-12, wherein the a consecutive cycles of base extension reactions comprise the designated cycle of base extension reaction, the n preceding cycles of base extension reactions, and the m succeeding cycles of base extension reactions, wherein n and m are both natural numbers greater than or equal to 1.

27. The method according to claim 26, wherein n is less than or equal to 5, and/or
m is less than or equal to 5.

28. The method according to claim 27, wherein n is 1, 2, or 3, and/or
m is 1, 2, or 3.

29. The method according to any one of claims 26-28, comprising, prior to determining the first sequencing information based on intensity features of combined bases comprising the base of the designated cycle:
mapping the coordinates of the second spots in the spot set of the sequencing template to the image corresponding to the designated cycle of base extension reaction, so as to determine the first spot in the image;
correcting, based on the initial intensity of the first spot in the image, to give the corrected intensity; and
determining the base type with the maximum brightness intensity in the four types of bases in the first spot based on the corrected intensity, and determining the base type as a predicted base type of the designated cycle of base extension reaction.

30. The method according to claim 29, wherein determining a base type of the designated cycle of base extension reaction based on the first sequencing information and a basecall model comprises:
determining the basecall model based on the number of consecutive bases of the a consecutive cycles of base extension reactions;
inputting the first sequencing information into the basecall model, outputting a model base combination, and determining the model base combination as the base combination corresponding to the a consecutive cycles of base extension reactions; and
correcting the predicted base type based on the base combination to give the base type of the designated cycle of base extension reaction.

31. The method according to any one of claims 26-28, comprising, prior to determining the first sequencing information based on intensity features of combined bases comprising the base of the designated cycle:
mapping the coordinates of the second spots in the spot set of the sequencing template to the images corresponding to the a consecutive cycles of base extension reactions, so as to determine the position of the first spot in each of the images;
correcting, based on the initial intensity of the first spot in each of the images, to give the corrected intensity;
determining the base type with the maximum brightness intensity in the four types of bases in the first spot based on the corrected intensity, and determining the base type as a predicted base type corresponding to the first spot; and
determining predicted combined base types based on the predicted bases corresponding to the designated cycle of base extension reaction, the n preceding cycles of base extension reactions, and the m succeeding cycles of base extension reactions.

32. The method according to claim 31, wherein determining a base type of the designated cycle of base extension reaction based on the first sequencing information and a basecall model comprises:
determining the basecall model based on the number of consecutive bases of the a consecutive cycles of base extension reactions;
inputting the first sequencing information into the basecall model, and outputting the base combination; and
correcting the predicted combined base types based on the base combination, so as to determine the base combination type as the base types of the a consecutive cycles of base extension reactions.

33. An apparatus for base calling, comprising:
a first sequencing information confirmation module, configured for determining a first sequencing information based on intensity features of a first spot in images corresponding to a consecutive cycles of base extension reactions comprising a designated cycle of base extension reaction, wherein a is a natural number greater than or equal to 1; and
a designated-cycle base type confirmation module, configured for determining a base type of the designated cycle of base extension reaction based on the first sequencing information and a basecall model, wherein the basecall model is determined based on a second sequencing information corresponding to the a consecutive cycles of base extension reactions in a training sample and base type of at least one cycle of base extension reaction in the a consecutive cycles of base extension reactions, and the second sequencing information comprises the first sequencing information.

34. The apparatus according to claim 33, wherein the a consecutive cycles of base extension reactions comprise a designated cycle of base extension reaction requiring base calling, and at least one of the n preceding cycles of base extension reactions and the m succeeding cycles of base extension reactions, wherein n is an integer greater than or equal to 0, and m is an integer greater than or equal to 0.

35. The apparatus according to claim 34, wherein the first sequencing information comprises at least one of the following features or a combination thereof, or a feature formed by dimensionality reduction for a combination of more than one of the following features:
the ratio of an intensity corresponding to a base channel with the maximum brightness to the total intensity in the four base channels at the coordinate position corresponding to the first spot in the image acquired in the designated cycle of base extension reaction;
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} preceding cycle to the n^{th} preceding cycle of base extension reactions;
the intensity feature of the A base channel at the coordinate position corresponding to the first spot in the image acquired in each cycle of base extension reaction from the 1^{st} succeeding cycle to the m^{th} succeeding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} preceding cycle to the n^{th} preceding cycle of base extension reactions;
the intensity feature of the C base channel at the coordinate position corresponding to the first spot in the image acquired in each cycle of base extension reaction from the 1^{st} succeeding cycle to the m^{th} succeeding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} preceding cycle to the n^{th} preceding cycle of base extension reactions;
the intensity feature of the G base channel at the coordinate position corresponding to the first spot in the image acquired in each cycle of base extension reaction from the 1^{st} succeeding cycle to the m^{th} succeeding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} preceding cycle to the n^{th} preceding cycle of base extension reactions;
the intensity feature of the T/U base channel at the coordinate position corresponding to the first spot in the image acquired in each cycle of base extension reaction from the 1^{st} succeeding cycle to the m^{th} succeeding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

36. The apparatus according to any one of claims 33-35, further comprising:
an intensity feature determination module, configured for mapping the coordinates of second spots in a spot set of a sequencing template to each of the images, so as to determine the first spot in the images and
determining the intensity features of the first spot in the images corresponding to the a consecutive cycles of base extension reactions based on the first spot in each of the images.

37. The apparatus according to claim 36, wherein the intensity feature determination module is further configured for:
mapping the coordinates of the second spots in the spot set of the sequencing template to each of the images, so as to determine a position corresponding to the coordinates of the second spot in the image, and determining a spot corresponding to the position as the first spot.

38. The apparatus according to any one of claims 33-35, wherein the intensity feature comprises any one of an initial intensity and a corrected intensity.

39. The apparatus according to claim 38, wherein the intensity feature comprises the initial intensity, and the module for confirming the intensity features of the first spot in the images corresponding to the a consecutive cycles of base extension reactions is configured for:
acquiring the images obtained from the a consecutive cycles of base extension reactions, extracting original intensities of the A base channel, the C base channel, the G base channel, and the T/U base channel at the first spot in the images, and confirming that the original intensities of the A base channel, the C base channel, the G base channel, and the T/U base channel are the initial intensities of the A base channel, the C base channel, the G base channel, and the T/U base channel at the first spot, respectively.

40. The apparatus according to claim 38, wherein the corrected intensity comprises at least one of a background-corrected intensity, a crosstalk-corrected intensity, and a reaction asynchrony-corrected intensity.

41. The apparatus according to claim 40, wherein the module for confirming the intensity features of the first spot in the images corresponding to the a consecutive cycles of base extension reactions is further configured for:
acquiring the images obtained from the a consecutive cycles of base extension reactions, extracting original intensities of the A base channel, the C base channel, the G base channel, and the T/U base channel at the first spot in the images, and confirming that the original intensities of the A base channel, the C base channel, the G base channel, and the T/U base channel are the initial intensities of the A base channel, the C base channel, the G base channel, and the T/U base channel at the first spot, respectively; and
confirming the corrected intensity by performing at least one of a background-corrected intensity, a crosstalk-corrected intensity, and a reaction asynchrony-corrected intensity based on the initial intensities.

42. The apparatus according to any one of claims 33-41, wherein the designated-cycle base type confirmation module is further configured for:
determining a base combination type corresponding to the a consecutive cycles of base extension reactions;
and
inputting the first sequencing information into the basecall model matching the base combination type, outputting a base combination corresponding to the a consecutive cycles of base extension reactions, and determining the base type of the designated cycle of base extension reaction based on the base combination.

43. The apparatus according to claim 42, wherein an establishment module of the basecall model is configured for:
acquiring a training sample set, wherein each sample in the training sample set is labeled with a feature value and a target value, the feature value is a second sequencing information determined based on the base combination type, and the target value is the base type corresponding to the designated cycle of base extension reaction in the base combination type; and
performing machine learning modeling on each sample in the training sample set based on a specific model structure to give the basecall model.

44. The apparatus according to claim 43, wherein the specific model structure is the lightGMB model.

45. The apparatus according to any one of claims 33-44, wherein the a consecutive cycles of base extension reactions comprise the designated cycle of base extension reaction requiring base calling and the n preceding cycles of base extension reactions, wherein n is a natural number greater than or equal to 1.

46. The apparatus according to claim 45, wherein n is less than or equal to 5.

47. The apparatus according to claim 46, wherein n is 1, 2, or 3.

48. The apparatus according to any one of claims 45-47, wherein the first sequencing information comprises at least one of the following features or a combination thereof, or a feature formed by dimensionality reduction for a combination of more than one of the following features:
the ratio of an intensity corresponding to a base channel with the maximum brightness to the total intensity in the four base channels at the coordinate position corresponding to the first spot in the image acquired in the designated cycle of base extension reaction;
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} preceding cycle to the n^{th} preceding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} preceding cycle to the n^{th} preceding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} preceding cycle to the n^{th} preceding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} preceding cycle to the n^{th} preceding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

49. The apparatus according to claim 48, wherein the designated-cycle base type confirmation module is further configured for:
determining the basecall model based on the number of consecutive bases of the a consecutive cycles of base extension reactions;
inputting the first sequencing information into the basecall model, outputting the base combination, and determining the base combination as the base combination corresponding to the a consecutive cycles of base extension reactions; and
determining the base type of the designated cycle of base extension reaction based on the base combination.

50. The apparatus according to any one of claims 45-49, wherein the base combination corresponding to the a consecutive cycles of base extension reactions is a double-base combination comprising a base of the designated cycle of base extension reaction and a base of the preceding cycle of base extension reaction.

51. The apparatus according to claim 50, wherein the first sequencing information comprises: the ratio of an intensity corresponding to a base channel with the maximum brightness to the total intensity in the four base channels at the coordinate position corresponding to a valid spot in the image acquired in the designated cycle of base extension reaction.

52. The apparatus according to any one of claims 33-44, wherein the a consecutive cycles of base extension reactions comprise the designated cycle of base extension reaction requiring base calling and the m succeeding cycles of base extension reactions, wherein m is a natural number greater than or equal to 1.

53. The apparatus according to claim 52, wherein m is less than or equal to 5.

54. The apparatus according to claim 53, wherein m is 1, 2, or 3.

55. The apparatus according to any one of claims 52-54, wherein the first sequencing information comprises at least one of the following features or a combination thereof, or a feature formed by dimensionality reduction for a combination of more than one of the following features:
the ratio of an intensity corresponding to a base channel with the maximum brightness to the total intensity in the four base channels at the coordinate position corresponding to the first spot in the image acquired in the designated cycle of base extension reaction;
the total number of the first spots in the image acquired in the designated cycle of base extension reaction;
the intensity feature of the A base channel at the coordinate position corresponding to the first spot in the image acquired in the designated cycle of base extension reaction;
the intensity feature of the A base channel at the coordinate position corresponding to the first spot in the image acquired in each cycle of base extension reaction from the 1^{st} succeeding cycle to the m^{th} succeeding cycle of base extension reactions;
the intensity feature of the C base channel at the coordinate position corresponding to the first spot in the image acquired in the designated cycle of base extension reaction;
the intensity feature of the C base channel at the coordinate position corresponding to the first spot in the image acquired in each cycle of base extension reaction from the 1^{st} succeeding cycle to the m^{th} succeeding cycle of base extension reactions;
the intensity feature of the G base channel at the coordinate position corresponding to the first spot in the image acquired in the designated cycle of base extension reaction;
the intensity feature of the G base channel at the coordinate position corresponding to the first spot in the image acquired in each cycle of base extension reaction from the 1^{st} succeeding cycle to the m^{th} succeeding cycle of base extension reactions;
the intensity feature of the T/U base channel at the coordinate position corresponding to the first spot in the image acquired in the designated cycle of base extension reaction;
the intensity feature of the T/U base channel at the coordinate position corresponding to the first spot in the image acquired in each cycle of base extension reaction from the 1^{st} succeeding cycle to the m^{th} succeeding cycle of base extension reactions;
the ratio of an intensity corresponding to a base channel with the maximum intensity to an intensity corresponding to a base channel with the second maximum intensity in the four base channels at the coordinate position corresponding to the first spot in the image acquired in the designated cycle of base extension reaction; and
the standard deviation of the intensities corresponding to the four base channels at the coordinate position corresponding to the first spot in the image acquired in the designated cycle of base extension reaction.

56. The apparatus according to claim 55, further comprising:
a first predicted base type, configured for mapping the coordinates of the second spots in the spot set of the sequencing template to each of the images, so as to determine the first spot in the images;
correcting, based on the initial intensity of the first spot in each of the images, to give the corrected intensity;
and
determining the base type with the maximum brightness intensity in the four types of bases in the first spot based on the corrected intensity, and determining the base type as a predicted base type of the designated cycle of base extension reaction.

57. The apparatus according to claim 56, wherein the designated-cycle base type confirmation module is further configured for:
determining the basecall model based on the number of consecutive bases of the a consecutive cycles of base extension reactions;
inputting the first sequencing information into the basecall model, outputting the base combination, and determining the base combination as the base combination corresponding to the a consecutive cycles of base extension reactions; and
correcting the predicted base type based on the base combination.

58. The apparatus according to any one of claims 33-44, wherein the a consecutive cycles of base extension reactions comprise the designated cycle of base extension reaction, the n preceding cycles of base extension reactions, and the m succeeding cycles of base extension reactions.

59. The apparatus according to claim 58, wherein n is less than or equal to 5, and/or m is less than or equal to 5.

60. The apparatus according to claim 59, wherein n is 1, 2, or 3, and/or
m is 1, 2, or 3.

61. The apparatus according to any one of claims 58-60, further comprising:
a second predicted base type determination module, configured for mapping the coordinates of the second spots in the spot set of the sequencing template to the image corresponding to the designated cycle of base extension reaction, so as to determine the first spot in the image;
correcting, based on the initial intensity of the first spot in the image, to give the corrected intensity; and
determining the base type with the maximum brightness intensity in the four types of bases in the first spot based on the corrected intensity, and determining the base type as a predicted base type of the designated cycle of base extension reaction.

62. The apparatus according to claim 61, wherein the designated-cycle base type determination module is further configured for:
determining the basecall model based on the number of consecutive bases of the a consecutive cycles of base extension reactions;
inputting the first sequencing information into the basecall model, outputting a model base combination, and determining the model base combination as the base combination corresponding to the a consecutive cycles of base extension reactions; and
correcting the predicted base type based on the base combination to give the base type of the designated cycle of base extension reaction.

63. The apparatus according to any one of claims 58-60, further comprising:
a predicted combined base type determination module, configured for mapping the coordinates of the second spots in the spot set of the sequencing template to the images corresponding to the a consecutive cycles of base extension reactions, so as to determine the position of the first spot in each of the images;
correcting, based on the initial intensity of the first spot in each of the images, to give the corrected intensity;
determining the base type with the maximum brightness intensity in the four types of bases in the first spot based on the corrected intensity, and determining the base type as a predicted base type corresponding to the first spot; and
determining predicted combined base types based on the predicted bases corresponding to the designated cycle of base extension reaction, the n preceding cycles of base extension reactions, and the m succeeding cycles of base extension reactions.

64. The apparatus according to claim 63, wherein the designated-cycle base type confirmation module is further configured for:
determining the basecall model based on the number of consecutive bases of the a consecutive cycles of base extension reactions;
inputting the first sequencing information into the basecall model, and outputting the base combination; and
correcting the predicted combined base types based on the base combination, so as to determine the base combination type as the base types of the a consecutive cycles of base extension reactions.

65. An electronic device, comprising:
one or more processors; and
a storage apparatus, configured for storing one or more programs, wherein
when the one or more programs are executed by the one or more processors, the one or more processors implement the method for base calling according to any one of claims 1-32.

66. A storage medium comprising a computer-executable instruction, wherein the computer-executable instruction is configured for executing the method for base calling according to any one of claims 1-32 when executed by a computer processor.
